# EUROPEAN PATENT APPLICATION

(11) **EP 4 559 474 A1**
(43) Date of publication of application: **28.05.2025**
(21) Application number: 23842282.8
(22) Date of filing: 18.07.2023
(51) Int. Cl.: A61K 38/19, A61K 47/60, A61K 47/64, A61P 35/00

(54) **TUMOR MICROENVIRONMENT-ACTIVATED CYTOKINE AND USE THEREOF**

(30) Priority: 18.07.2022 CN 202210844222
(71) Applicant: Yafei Shanghai Biology Medicine Science & Technology Co., Ltd., Shanghai 201203 (CN)
(72) Inventor: ZHANG, Rui, Shanghai 201203 (CN); CHEN, Tao, Shanghai 201203 (CN); LIU, Yuan, Shanghai 201203 (CN); LIU, Chen, Shanghai 201203 (CN)
(74) Representative: IPLodge bv
(86) International application number: PCT/CN2023/107809
(87) International publication number: WO 2024/017219

(57) **Abstract**

Disclosed are a tumor microenvironment-activated cytokine IFNα and use thereof. After cysteine mutation of IFN-α, IFN-α is joined to a substrate and a mask body that can be recognized and cleaved by substances highly expressed by tumors to form a biomolecular conjugate. The INF-α activity of the biomolecular conjugate in normal tissues is hindered, not causing toxicity to normal tissues. The INF-α activity is activated for effective tumor inhibition in tumor environments.

## Description

The present disclosure claims the benefit of priority to Patent Application CN 202210844222.6, named tumor microenvironment-activated cytokine and use thereof and filed July 18, 2022, with its entire content incorporated herein by reference.

### FIELD OF THE DISCLOSURE

The present disclosure refers to the field of biochemistry. More specifically, the present disclosure refers to tumor microenvironment-activated cytokine and use thereof.

### BACKGROUND OF THE DISCLOSURE

Cytokines are molecular messengers that allow immune cells to communicate with each other, generating a coordinated, robust and self-limiting response to target antigens. Although the immune system often communicates with each other by direct intercellular interactions, cytokine secretions enables immune signals to spread rapidly in a multifaceted and efficient manner.

In recent years, several cytokines, comprising GM-CSF, IL-7, IL-12, IL-15, IL-18 and IL-21, have entered clinical trials for patients with advanced cancer. Based on potent anti-tumor activities of several pro-inflammatory cytokines discovered in animal models, recombinant interferon-α and interleukin-2 have been approved for clinical trials in the treatment of various malignancies, even though their efficacy still requires improvement.

Interferons are classified according to their ability to bind to specific receptors known as Type I, Type II and the recently described Type III IFN receptors. Although high-dose IFNα is the most commonly used regimen for providing adjuvant therapy to melanoma patients, therapies with better therapeutic indexes are urgently needed and several alternative regimens are currently under investigation. Experience with the use of IFN has led to established guidelines for recognizing and managing toxicity and side effects. Toxic features of IFNα are typically dose-dependent and most side effects can be controlled without discontinuing treatment. Systemic symptoms, comprising fever, fatigue, headache, gastrointestinal symptoms and myalgia are common and may occur in 80% or more patients. IFNα can also elevate liver enzymes in some patients, particularly during high-dose intravenous administration, at which point frequent monitoring is necessary, and treatment should be continued or the dose should be reduced in patients with elevated liver enzymes. Therefore, reducing the dosage of IFNα and improving the administration effect of IFNα are urgent problems to be solved in this field.

### SUMMARY OF THE DISCLOSURE

The aim of the present disclosure is to provide a tumor microenvironment-activated cytokine IFNα and use thereof.

In the first aspect, the present disclosure provides a biomolecular conjugate of cytokine IFNα with the following structure:

**R1-AAN-PABC-R3-S-Cys-IFNα**

wherein,
Cys represents a cysteine residue introduced through single point mutation on the surface of IFNα;
S represents a sulfur atom on the cysteine residue;
IFNα has an amino acid sequence shown in SEQ ID NO: 1;
R1 is a group that prevents IFNα mutants from binding to its ligand or receptor;
AAN represents a tripeptide, wherein A is alanine, N is aspartic acid and PABC is para-aminobenzyl carbamate;
R3 is a linking arm covalently conjugated to the sulfur atom of the cysteine introduced by the single mutation of IFNα via an EMC group; The function of R3 is to remain attached to the cytokine after AAN cleavage, allowing R3-S-Cys-IFNα partially restores, fully recovers, or even enhances the affinity to the IFNα receptor.

In one or more embodiments, in the biomolecular conjugate, R1 is selected from (but are not limited to) polyethylene glycol with a molecular weight of 44 to 132000, such as polyethylene glycol with a molecular weight of 1000 to 50000, 3,000 to 80000 or 10000 to 60000;

In one or more embodiments, the IFNα mutant is a mutant obtained by single point mutation on INF-α, wherein the IFNα mutant has mutations of Arg149, Ala145, His57, Gln61, Phe64 or Ser68, Phe27, Ser152, Arg162 residues to Cys, according to the amino acid sequence of SEQ ID NO: 1.

In one or more embodiments, in the biomolecular conjugate, R1 is selected from:

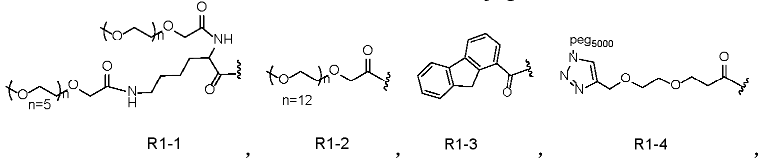

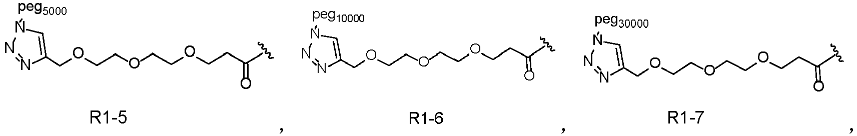

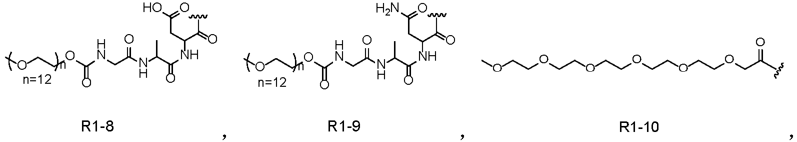

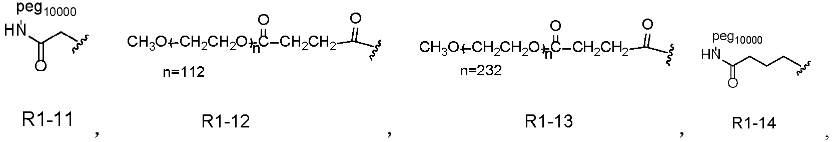

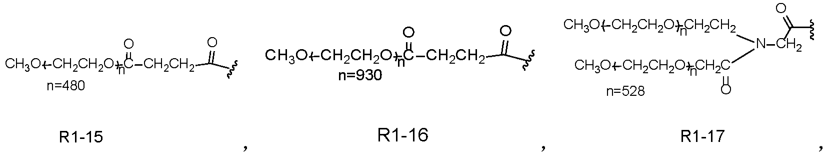

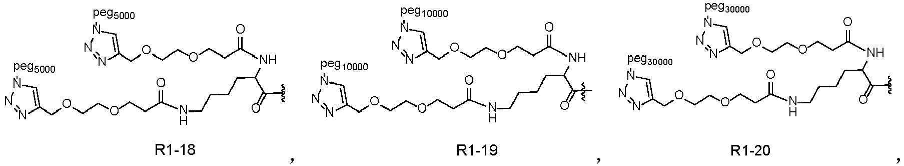

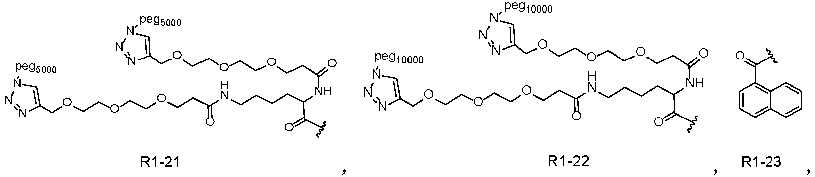

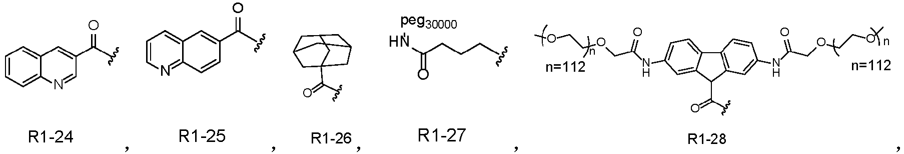

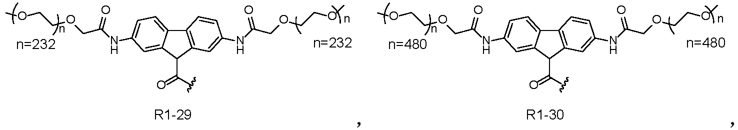

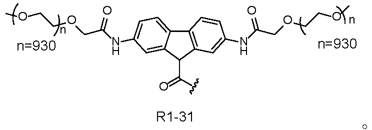

In one or more embodiments, in the biomolecular conjugate, R3 is selected from (but are not limited to):

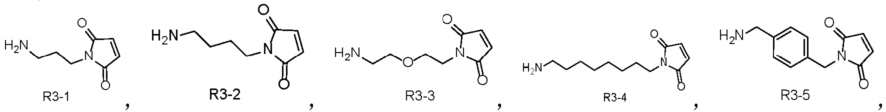

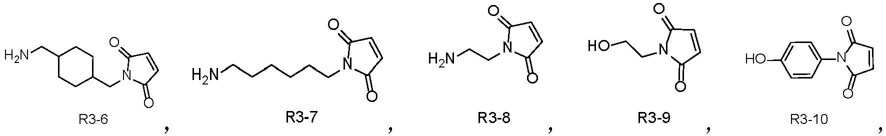

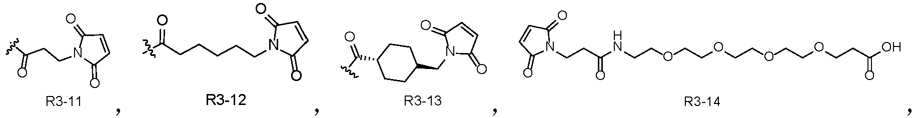

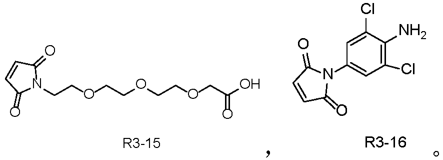

In one or more embodiments, in the biomolecular conjugate, R1-AAN-PABC-R3 has a structure selected from:

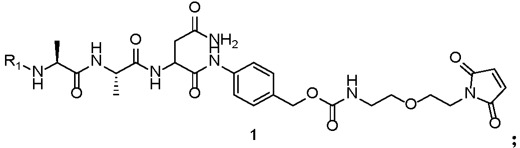

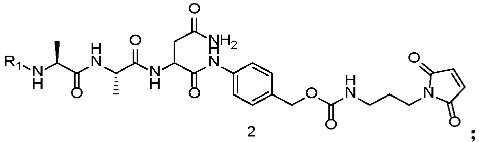

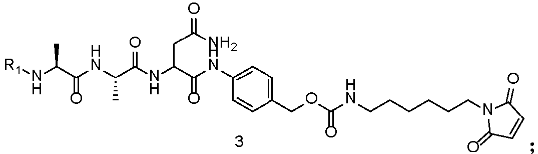

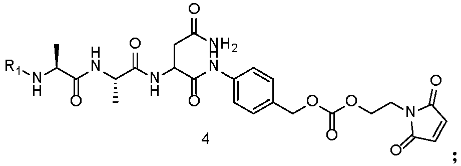

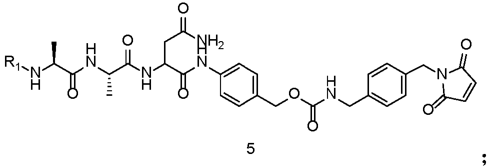

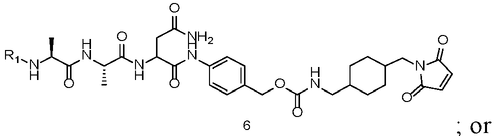

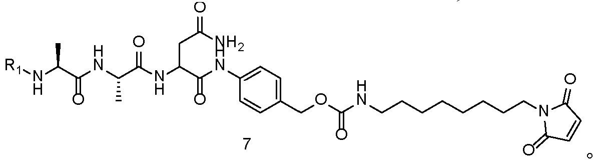

In another aspect, the present disclosure provides a use of the biomolecular conjugate for preparing a composition or drug kit for inhibiting tumors.

In another aspect, the present disclosure provides a use of the biomolecular conjugate for preparing a composition or drug kit for inhibiting tumors in combination with an anti-tumor antibody.

In another aspect, the present disclosure provides a composition or drug kit for inhibiting tumors, wherein the composition or kit comprises the biomolecular conjugate; preferably, the composition also comprises a pharmaceutically acceptable carrier.

In one or more embodiments, the composition or drug kit also comprises an anti-tumor antibody.

In one or more embodiments, the drug kit also comprises: an anti-tumor antibody or a composition comprising an anti-tumor antibody.

In one or more embodiments, the anti-tumor antibody is an anti-PD-1 antibody.

In one or more embodiments, the biomolecular conjugate and the anti-PD-1 antibody are at a mass ratio of 1: (0.05-50) (such as 1: 0.1, 1: 0.15, 1: 0.2, 1: 0.4, 1: 0.5, 1: 0.6, 1: 0.8, 1: 1, 1: 2, 1: 4, 1: 5, 1: 6, 1: 8, 1: 10, 1: 20, 1: 30, 1: 40); preferably 1: (0.2-8); more preferably 1: (0.4-5).

In one or more embodiments, dosage forms of the composition comprise (but are not limited to): injections, infusions, powders, tablets, capsules, etc.; preferably injections.

In one or more embodiments, the drug kit comprises a container, wherein the INF-α mutant and/or the anti-tumor antibody or a composition comprising the INF-α mutant and/or the anti-tumor antibody is placed in the container.

In one or more embodiments, the container comprises (but is not limited to): a syringe.

In one or more embodiments, the drug kit also comprises: instructions describing methods of inhibiting tumors.

In another aspect, the present disclosure provides a method of preparing activated INF-α in a tumor microenvironment, comprising: mutating INF-α at specific residues of the amino acid sequence thereof to Cys by single point mutation.

In another aspect, the present disclosure provides an IFNα mutant, wherein the mutant is obtained by single point mutation on INF-α and wherein the IFNα mutant has mutations of Arg 149, Ala145, His57, Gln61, Phe64 or Ser68, Phe27, Ser152, Arg162 residues to Cys, according to the amino acid sequence of SEQ ID NO: 1.

In another aspect, the present disclosure provides an isolated polynucleotide, wherein the polynucleotide encodes the INF-α mutant.

In another aspect, the present disclosure provides a vector, wherein it comprises the polynucleotide.

In another aspect, the present disclosure provides a genetically engineered host cell, wherein it comprises the vector, or the polynucleotide integrated into genome.

In another aspect, the present disclosure provides a method for preparing the INF-α mutant, comprising the steps of: (1) culturing the host cell to obtain a culture; and (2) isolating the INF-α mutant from the culture.

In another aspect, the present disclosure provides a use of the INF-α mutant for conjugating with **R1-AAN-PABC-R3** to form a biomolecular conjugate that inhibits tumors; wherein, R1 is a group that prevents IFNα mutants from binding to its ligand or receptor; AAN represents a tripeptide, wherein A is alanine, N is aspartic acid and PABC is para-aminobenzyl carbamate; R3 is a linking arm covalently conjugated to the sulfur atom of the cysteine introduced by the single mutation of IFNα via an EMC group; The function of R3 is to remain attached to the cytokine after AAN cleavage, allowing R3-S-Cys-IFNα partially restores, fully recovers, or even enhances the affinity to the IFNα receptor.

In one or more embodiments, inhibiting tumors comprises preventing, alleviating, and/or treating tumors.

In one or more embodiments, the anti-tumor antibody is a monoclonal antibody or a polyclonal antibody.

In one or more embodiments, the anti-PD-1 antibody is any antibody that specifically neutralizes/binds PD-1.

In one or more embodiments, the tumors comprise: melanoma, NSCLC, head and neck squamous cell carcinoma, urothelial carcinoma, classic Hodgkin lymphoma, gastric cancer, adenocarcinoma of esophagogastric junction, cervical cancer, B-cell lymphoma, hepatocellular carcinoma, Merkel cell carcinoma, renal cell carcinoma, primary liver cancer, small cell lung cancer.

Other aspects of the present disclosure will be apparent to those skilled in the art based on the disclosure herein.

### DESCRIPTION OF DRAWINGS

Figure 1. Effects of mutant IFN-α conjugates on cellular signaling after conjugation and activation.
Figure 2. Therapeutic effects of activated IFN-α in a tumor microenvironment on MC38 tumors.
Figure 3. Anti-tumor effects of the combination of an IFN-α conjugate with an anti-PD-1 antibody.

### DETAILED DESCRIPTION

After in-depth researches, the inventors have revealed an optimized modified INF-α, wherein the IFN-α is joined to a substrate and a mask body (R) that can be recognized and cleaved by substances highly expressed by tumors to form a biomolecular conjugate. The INF-α activity of the biomolecular conjugate in normal tissues is hindered, not causing toxicity to normal tissues. The INF-α activity is activated for effective tumor inhibition in tumor environments. The conjugate of the present disclosure enable inhibitory activity of INF-α highly focused in the tumor environment, thereby achieving an ideal anti-tumor effect.

### Terms

As used herein, unless otherwise specified, "IFNα mutant" and "mutant IFNα" are interchangeable terms referring to products obtained by mutating wild-type IFNα; preferably, the terms refer to proteins resulting from amino acid residue mutations at the following positions in wild-type IFNα (e.g., SEQ ID NO: 1): Arg149, Ala145, His57, Gln61, Phe64 or Ser68, Phe27, Ser152, Arg162. More preferably, the residues Arg149, Ala145, His57, Gln61, Phe64 or Ser68 are mutated to Cys.

As used herein, if it is necessary to represent wild-type IFNα, it is referred to as WT, the protein with the amino acid sequence shown in SEQ ID NO: 1.

As used herein, "recombinant" refers to proteins, genetic engineering vectors or cells obtained (or prepared in large quantities) by means of genetic engineering.

As used herein, "antibody" encompasses monoclonal antibodies, polyclonal antibodies, dimers, multimers, multispecific antibodies (for example, bispecific antibodies), and antibody fragments, provided they exhibit desired biological activity.

As used herein, "comprise", "contain" or "include" means that various components can be used together in the mixture or composition of the present disclosure. Therefore, the term "mainly consist of...... " and "consist of...... " are included in the term "comprise", "contain" or "include". All weight or volume percentages should sum to 100%.

As used herein, "inhibition", "downregulation", "weakening" or "reduction" indicates a statistically significant "inhibition", "downregulation", "weakening" or "reduction". For example, compared to the control group, "inhibition", "downregulation", "weakening" or "reduction" is significant; more specifically, for example, more than 20%, preferably more than 50%, more preferably more than 80% "inhibition", "downregulation", "weakening" or "reduction". In the present disclosure, unless otherwise stated, inhibiting tumors comprises preventing, alleviating, and/or treating tumors.

As used herein, an anti-PD-1 antibody is any antibody that specifically neutralizes/binds PD-1.

In the present disclosure, a "pharmaceutically acceptable" component is one that is suitable for use with humans and/or animals without undue adverse side effects (such as toxicity, irritation and allergic response), that is, a substance with a reasonable benefit risk ratio.

As used herein, a "pharmaceutically acceptable carrier" is a pharmaceutically acceptable solvent, suspending agent or excipient used to deliver an active ingredient of the present disclosure to an animal or human. A "pharmaceutically acceptable carrier" may be a liquid or a solid.

As used herein, an "effective amount" means an amount of an ingredient sufficient to produce desired reactions.

### INF-α mutants

The present disclosure provides an INF-α mutant, wherein Arg149, Ala145, His57, Gln61, Phe64 or Ser68, Phe27, Ser152, Arg162 residues of the amino acid sequence are mutated to Cys; preferably, the Arg149 residue is mutated to Cys.

INF-α mutants of the present disclosure can be the product of chemical synthesis or produced by recombinant methods from prokaryotic or eukaryotic hosts (eg, bacteria, yeast, higher plants, insects and mammalian cells).

The present disclosure also comprises fragments, derivatives and analogues of the INF-α mutants. As used herein, the terms "fragment", "derivative", and "analogue" refer to proteins that maintain essentially the same biological function or activity as the original INF-α mutant of the present disclosure. The protein fragments, derivatives or analogues of the present disclosure may be (i) proteins having one or more conservative or non-conservative amino acid residues (preferably conservative amino acid residues) substituted, and such substituted amino acid residues may or may not be encoded by the genetic code, or (ii) a protein having a substituent group in one or more amino acid residues, or (iii) a protein formed by an additional amino acid sequences fused to the protein sequence (such as leader sequence or secretory sequence or sequence used to purify the protein or proprotein sequence, or fused protein). According to the teaching herein, these fragments, derivatives and analogues belong to the common knowledge to those skilled in the art. However, it should be satisfied that: at least one of the mutations described above in this disclosure must be present in the amino acid sequences of the INF-α mutants and fragments, derivatives and analogs thereof, preferably, the mutation is a mutation of Arg149, Ala145, His57, Gln61, Phe64 or Ser68, Phe27, Ser152, Arg162 mutated to Cys corresponding to the amino acid sequence shown in SEQ ID NO: 1; preferably, Arg149 is mutated to Cys.

In the present disclosure, the "INF-α mutant" further comprises (but is not limited to): deletion, insertion and/or substitution of one or more (usually 1-20, preferably 1-10, more preferably 1-8, 1-5, 1-3 or 1-2) amino acids, and addition or deletion of one or several (usually within 20, preferably within 10, more preferably within 5) amino acids at the C-terminal and/or N-terminal. For example, in the art, substitution with amino acids of approaching or similar property generally does not alter the function of a protein. For another example, the addition of one or several amino acids to C-terminal and/or N-terminal also generally does not alter the function of a protein. The term also comprises functional fragments and functional derivatives of INF-α mutants. However, among these mutations, at least one of the mutations described above in this disclosure must be present, preferably, the mutation is a mutation of Arg149, Ala145, His57, Gln61, Phe64 or Ser68, Phe27, Ser152, Arg162 mutated to Cys corresponding to the amino acid sequence shown in SEQ ID NO: 1; preferably, Arg149 is mutated to Cys.

In the present disclosure, the term "INF-α mutant" further comprises (but is not limited to) derivatives retaining protein activity with at least 80%, preferably at least 85%, more preferably at least 90%, even more preferably at least 95%, for example at least 98%, at least 99% sequence identity with the amino acid sequence of the INF-α mutant. Likewise, in these derivatives, the mutations described above in this disclosure must be present, preferably, the mutation is a mutation of Arg149, Ala145, His57, Gln61, Phe64 or Ser68, Phe27, Ser152, Arg162 mutated to Cys corresponding to the amino acid sequence shown in SEQ ID NO: 1; preferably, Arg149 is mutated to Cys.

The present disclosure also provides analogs of the INF-α mutants. The difference between these analogs and the INF-α mutants may be differences in amino acid sequences, differences in modifications that do not affect the sequence, or both. These polypeptides include natural or induced genetic variants. Induced variants can be obtained by various techniques, such as random mutagenesis by radiation or exposure to mutagens, site-directed mutagenesis or other techniques known in molecular biology. Analogues also include analogs with residues that differ from natural L-amino acids (eg, D-amino acids), as well as analogs with non-naturally occurring or synthetic amino acids (eg, β, γ-amino acids). It should be understood that the polypeptides of the present disclosure are not limited to the representative proteins exemplified above. Modified (usually without changing the primary structure) forms comprise chemically derived forms of in-vivo or in-vitro polypeptides, for example acetylated or carboxylated forms. Modifications also include glycosylation, such as those resulting from glycosylation modifications either in the synthesis and processing of the protein or in further processing steps. This modification can be accomplished by exposing the polypeptide to enzymes for glycosylation, such as mammalian glycosylases or deglycosylases. The modified forms also include sequences with phosphorylated amino acid residues such as phosphotyrosine, phosphoserine, phosphothreonine. Polypeptides that were modified to improve their antiproteolytic properties or optimize the solubilization properties were also included.

The present disclosure also provides a polynucleotide sequence encoding the INF-α mutant or a conservative variant protein thereof in the present disclosure.

The polynucleotide of the present disclosure can be in the form of DNAs or RNAs. DNAs include cDNAs, genomic DNAs, or synthetic DNAs. DNAs can be single stranded DNAs or double stranded DNAs. DNAs can be coding or noncoding strand DNAs.

The polynucleotide encoding the mature protein of the mutant comprises: the coding sequence encoding only the mature protein; the coding sequence of the mature protein and various additional coding sequences; the coding sequence of the mature protein (and optional additional coding sequences) and non-coding sequence.

"Polynucleotide encoding a polypeptide" may include a polynucleotide encoding the polypeptide, or may include a polynucleotide further including additional coding and/or non-coding sequences.

The present disclosure also relates to vectors containing the polynucleotides of the present disclosure, as well as host cells produced by genetic engineering methods using the vectors or coding sequences of the INF-α mutants of the present disclosure, and methods for producing enzymes with the mutations of the present disclosure through recombinant technology.

Through conventional recombinant DNA technology, the polynucleotide sequences of the present disclosure can be used for expressing or producing recombinant INF-α mutants. Generally speaking, steps are as follows: (1) transforming or transducing an appropriate host cell with a polynucleotide encoding the INF-α mutants (or variants), or with a recombinant expression vector containing the polynucleotide; (2) culturing the host cells in a suitable medium; (3) isolating and purifying proteins from the culture medium or cells.

In the present disclosure, the polynucleotide sequence encoding the INF-α mutant protein can be inserted into a recombinant expression vector. Any plasmid and vector can be used as long as it can replicate and be stable in the hosts. An important characteristic of an expression vector is that it usually contains an origin of replication, a promoter, a marker gene, and translation control elements. Those skilled in the art are well known of methods for constructing expression vectors comprising DNA sequences encoded by the INF-α mutants and suitable transcription/translation control signals. The DNA sequence can be effectively ligated to an appropriate promoter in an expression vector to direct mRNA synthesis. The expression vector also includes a ribosome binding site for translation initiation and a transcription terminator. The expression vector preferably comprises one or more selectable marker genes to provide a phenotypic trait for selection of transformed host cells.

Vectors comprising the appropriate DNA sequences described above, together with appropriate promoter or control sequences, can be used to transform appropriate host cells so that they can express the protein.

In the present disclosure, the host cell may be a prokaryotic cell, such as a bacterial cell, preferably *Escherichia coli*; or a lower eukaryotic cell, such as a mold cell or yeast cell; or a higher eukaryotic cell, such as a plant cell. Representative examples comprise: *Escherichia coli*, *Bacillus subtilis*, *Streptomyces*, *Agrobacterium*; eukaryotic cells such as yeast, plant cells, etc.

Those skilled in the art will know how to select appropriate vectors, promoters, enhancers and host cells. The recombinant cells (host cells) established in the present disclosure can be cultured using conventional methods to express the polypeptides encoded by the genes of the present disclosure. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

When expressed, the INF-α mutants of the present disclosure can be expressed intracellularly, on the cell membrane or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods.

The optimized INF-α mutants of the present disclosure have multiple uses related to the properties of INF-α, including use as active molecules that inhibit tumors. It is understood that host cells expressing the mutants or their expression products (e.g., cleavage products or secreted products) can also be used as described above.

In a preferred embodiment, the INF-α mutant is used for conjugation with **R1-AAN-PABC-R3** to form biomolecular conjugates for tumor inhibition.

### Biomolecular conjugates

The INF-α mutants of the present disclosure can be prepared into biomolecular conjugates with the following structure:

**R1-L-IFNα mutant**

R1 is a protective group for the IFNα mutant, wherein it can be selected from any group capable of preventing the IFNα mutant from binding to its ligand or receptor, thereby avoiding interference from other molecules. For example, R1 prevents binding to its ligand or receptor before reaching a pathological microenvironment, such as a tumor microenvironment. Suitable R1 groups can be selected from polyethylene glycol (PEG), polyethylene glycol-C1-5 alkyl carbonyl, naphthyl carbonyl, quinolyl carbonyl, fluorenyl carbonyl or adamantyl carbonyl.

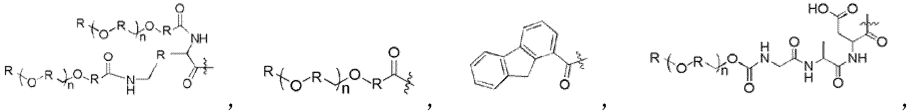

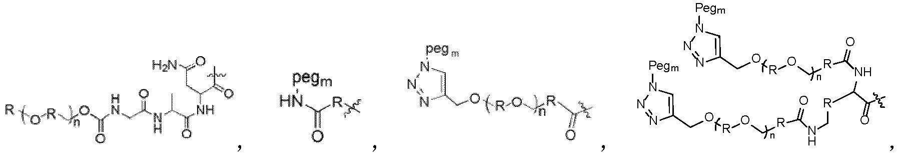

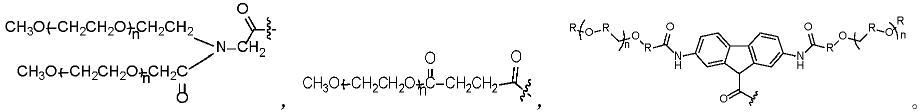

In the above structural formula, each R group is independently a C1-4 alkyl group; each n is independently an integer in the range of 1 to 30000, such as integers in the ranges of 1 to 15000, 1 to 5000, 1 to 2000, 1 to 300, 1 to 150, 1 to 50, or 1 to 20, or specifically 3 to 12; polyethylene glycol or Pegₘ represents polyethylene glycol with a molecular weight of 44 to 132000, such as polyethylene glycol with a molecular weight of 1000 to 50000 or 10000 to 30000; m represents the molecular weight of the polyethylene glycol; wavy line indicates the position that R1 is connected to L.

In some embodiments, R1 can be selected from:

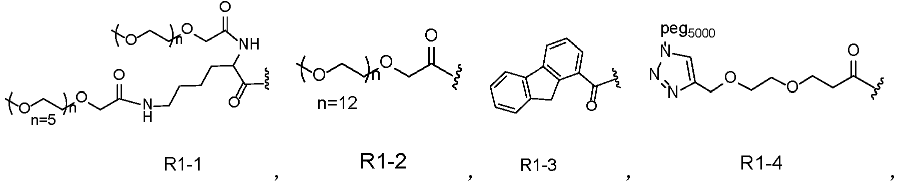

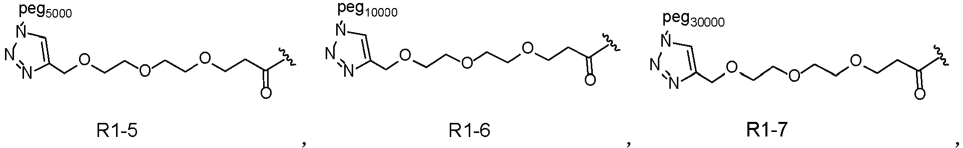

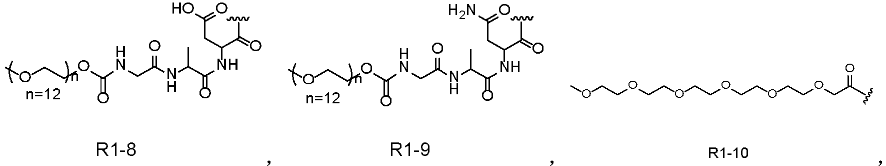

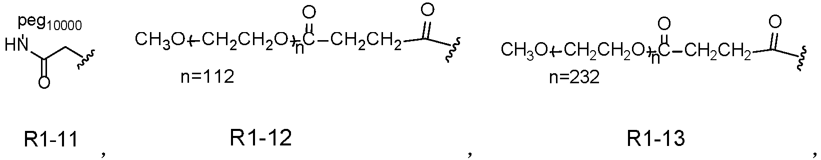

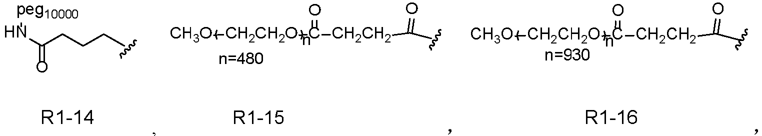

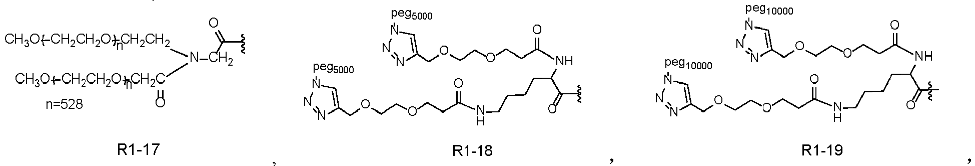

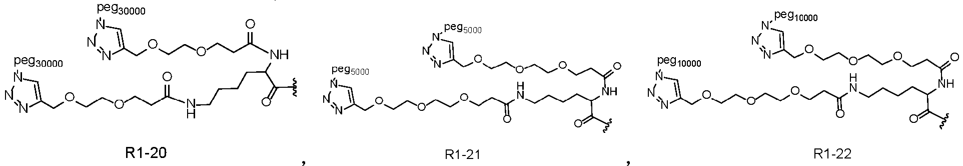

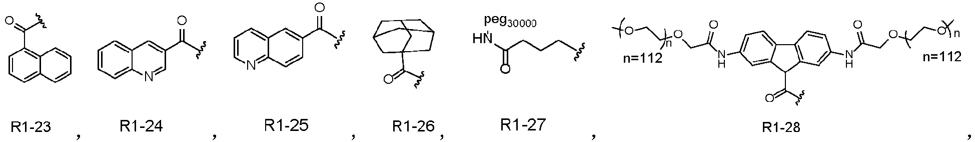

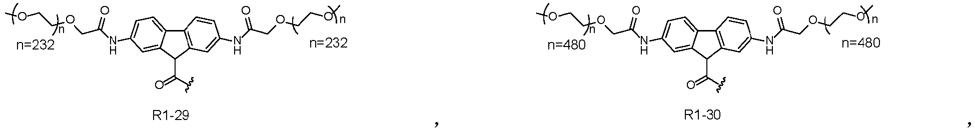

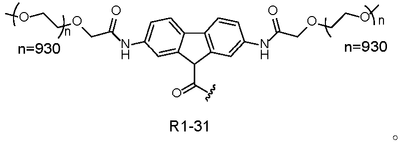

In the present disclosure, L is the linker; preferably, L comprises L1 and L2; preferably, L1 is AA-PABC and L2 is R3, wherein AA represents a dipeptide or tripeptide fragment (i.e., a fragment formed by 2-3 amino acids linked via peptide bonds), preferably Ala-Ala-Asn (alanine-alanine-asparagine) and PABC is p-aminobenzyl carbamate.

In the present disclosure, L2 can also be a cleavable linker arm. It may be a peptide that can be activated by proteases, proteinases or peptidases. In the present disclosure, the proteases, proteinases or peptidases can be those present in pathological microenvironments. For example, the protease may comprise cysteine proteases, aspartic proteases, glutamic proteases, threonine proteases, gelatinases, metalloproteases or asparaginyl endopeptidases. In certain embodiments, L2 can also be cleaved by at least one of the enzymes selected from asparaginyl endopeptidase (Legumain), cathepsin B, cathepsin C, cathepsin D, cathepsin E, cathepsin K, cathepsin L, kallikrein, hK1, hK10, hK15, plasmin, collagenase, type IV collagenase, astacin, factor Xa, chymotrypsin-like protease, trypsin-like protease, elastase-like protease, subtilisin-like protease, actinidain, bromelain, calpain, caspase, caspase-3, Mirl-CP, papain, HIV-1 protease, HSV protease, CMV protease, rennin, pepsin, matriptase, legumain, plasmepsin, nepenthesin, metalloexopeptidase, metalloendopeptidase, matrix metalloproteinases (MMPs), MMP1, MMP2, MMP3, MMP8, MMP9, MMP10, MMP11, MMP12, MMP13, MMP14, ADAM10, ADAM12, urokinase plasminogen activator (uPA), enteropeptidase, prostate-specific antigen (PSA, hK3), interleukin-1β converting enzyme, thrombin, fibroblast activation protein (FAP-a), transmembrane peptidase (meprin), granzyme, dipeptidyl peptidase, and dipeptidyl peptidase IV (DPPIV/CD26). In a preferred embodiment, the present disclosure relates to asparaginyl endopeptidase, primarily expressed and secreted by tumor cells in the tumor microenvironment. Tumor-associated macrophages (M2) also differ from monocytes and inflammatory macrophages (M1) through the expression of asparagine endopeptidase. In the present disclosure, a polypeptide can be a substrate for a proteolytic enzyme and can be recognized and cleaved by a proteolytic enzyme. L2 of the present disclosure can be represented by -L2a-, -L2b-, -L2a-N-, -L2a-D-, -L2a-AAN-, -L2a-AAD- or -L2a-L2b-; wherein L2a is a peptide cleavable by one or more proteases at an amide bond; L2b is a peptide that can form a carbamate with PABC via an amino group in its side chain and the carbamate can be cleaved by one or more proteases; A is alanine; N is asparagine, the amino group in its side chain forms carbamate with nearby groups and the carbamate can be cleaved by asparagine endopeptidase; D is asparagine acid, with its side chain amino group forming a carbamate with PABC that can be cleaved by granzyme B. L2a and L2b can be linked by forming an amide bond. Following cleavage by asparaginyl endopeptidase, granzyme B cleaves the bond (e.g., carbamate) between L2 and PABC, leading to a rapid self-release of PABC.

In some embodiments, suitable peptides activated by proteases may comprise tripeptides. Any substrate peptide known to be recognized and cleaved (activated) by proteases in pathological microenvironments can be used as L2 in the present disclosure. Such peptides have structures disclosed in WO 2016/026458 with its entire contents incorporated by reference in the present disclosure. In certain embodiments, in tripeptide structures suitable for the present disclosure, the amino acid residue connected to R1 can be selected from Ala, Thr, Val and Ile, the middle amino acid residue can be selected from Ala, Thr, Val and Asn, and the third amino acid residue can be selected from Asn and Asp. Usually, L connects to R1 via the amino group of its amino acid residue in the form of an amide, ester, carbamate, urea or hydrazone and connects to PABC via the carboxyl group of its amino acid residue in the form of an amide, ester, carbamate, urea, or hydrazone. In some preferred embodiments of the present disclosure, the tripeptide is selected from Ala-Ala-Asn and Ala-Ala-Asp. Ala-Ala-Asn can be recognized and cleaved by asparagine endopeptidase and Ala-Ala-Asp can be recognized and cleaved by granzymes.

A suitable R3 can be expressed as:

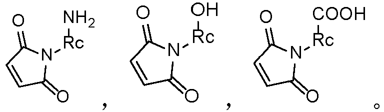

Wherein R_{c} is selected from C₁₋₁₂ alkyl, C₁₋₁₂ oxyalkyl-C₁₋₁₂ alkyl, C₁₋₁₂ alkyl-C₃₋₈ cycloalkyl, (C₁₋₄ alkyl- O) ₚ-C_{1 -12} alkyl, C₁₋₁₂ alkylcarbonylamino-(C₁₋₄ alkyl-O)p-C₁₋₁₂ alkyl, phenyl-C₁₋₁₂ alkyl, C₃₋₈ cycloalkyl, C₁₋₁₂ alkyl-C₃₋₈ cycloalkyl-C₁₋₁₂ alkyl and C₁₋₁₂ alkyl-phenyl-C₁₋₁₂ alkyl, wherein phenyl group can be one or two optionally substituted phenyl groups and the substituted group can be halogen atoms;
In some examples of the present disclosure the specific structure of R3 may be selected from:

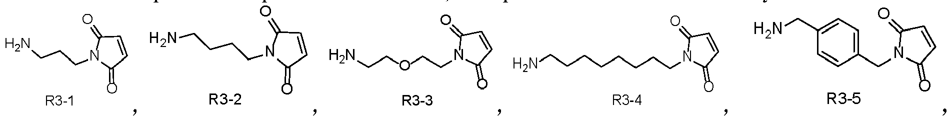

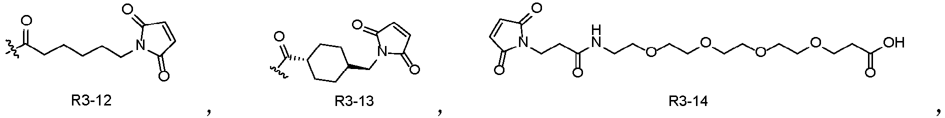

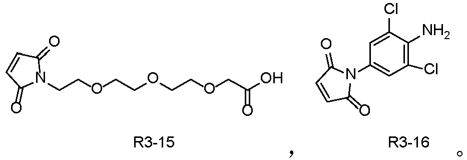

In the disclosed conjugates, R3 can form a covalent bond with the sulfur (S) of cysteine at specific sites of the IFNα mutant. R1 can be cleaved by proteases from the structure **R1-AAN-PABC-R3-S-**Cys-IFNα, releasing PABC-R3-S-Cys-IFNα. Subsequently, PABC spontaneously detaches, releasing R3-S-Cys-IFNα mutant.

It should be understood that in the present disclosure, the wavy line used in each indicated formula represents the site of connection between the part containing the wavy line and the rest of the molecule.

The conjugates disclosed herein, such as **R1-AAN-PABC-R3** and **R1-AAN-PABC-R3-S-**Cys-IFNα, can be synthesized using methods well-known in this field.

### Compositions

The present disclosure also comprises compositions comprising the disclosed biomolecular conjugates (e.g., pharmaceutical compositions) that comprise the conjugates as described herein. The pharmaceutical composition may further comprise a pharmaceutically acceptable carrier. The carrier may be any pharmaceutically acceptable carrier or excipient, varying with the dosage form and mode of administration. Pharmaceutically acceptable carriers are typically safe and non-toxic, and may include any known substances used in the pharmaceutical industry for formulating drug compositions, such as fillers, diluents, coagulants, binders, lubricants, auxiliaries, glidants, stabilizers, colorants, wetting agents, disintegrants, and so on. Suitable pharmaceutically 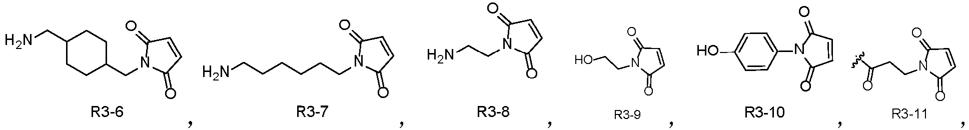 acceptable carriers comprise sugars, such as lactose or sucrose, mannitol or sorbitol; cellulose preparations and/or calcium phosphates, such as tricalcium phosphate or dicalcium phosphate; starch, including corn starch, wheat starch, rice starch, potato starch, gelatin, tragacanth, methylcellulose, hydroxypropylmethylcellulose, sodium carboxymethylcellulose and/or polyvinylpyrrolidone; silicon dioxide, talc, stearic acid or the salts thereof, such as magnesium stearate or calcium stearate; and/or polyethylene glycol, etc. When selecting a pharmaceutically acceptable carrier, a major consideration is the mode of administration of the pharmaceutical dosage form. This is well known in the art.

Pharmaceutical compositions may comprise a therapeutically or prophylactically effective amount of the conjugate. Specific effective amount depends on various factors, such as the specific disease to be treated, the patient's physical condition such as weight, age and sex, the duration of treatment, co-administered treatments (if any) and the specific formulation used. Generally, an "effective amount" as referred to herein is a conventional amount of the biomolecule. However, in some embodiments, the therapeutically or prophylactically effective amount of the conjugate in the pharmaceutical composition disclosed herein may be lower than conventional amounts of the biomolecule but may yield better therapeutic or prophylactic outcomes, as the biomolecule is protected by a masking group before reaching the pathological microenvironment for binding to its ligand or receptor.

The pharmaceutical compositions of this disclosure can be formulated into various suitable dosage forms, including but not limited to tablets, capsules, injections, etc., and can be administered via any appropriate route to achieve the intended purpose. For example, it can be administered parenterally, subcutaneously, intravenously, intramuscularly, intraperitoneally, transdermally, orally, intrathecally, intracranially, intranasally or topically. The dosage of the drug may depend on the age, health condition, and weight of the patient, concurrent therapies, treatment frequency, and other factors. The pharmaceutical compositions of the present disclosure can be administered to any subject in need, such as mammals, especially humans.

In tumor patients, tumor cells carrying tumor antigens or antigen-presenting cells (APCs) partially or completely suppress the host's immune response against the tumor through interaction with T cells. In some embodiments, the disclosed conjugates are activated and released in the pathological microenvironment by proteases, particularly asparaginyl endopeptidases or granzymes, or under acidic conditions.

Therefore, the disclosed conjugates can effectively penetrate the individual's immune barrier, reach the pathological microenvironment, and then be activated and released therein. As a result, they can selectively promote proliferation or cytotoxic effects of T cells or other cells in the tumor microenvironment, thereby achieving low autoimmunity and high efficacy.

The biomolecular conjugates disclosed herein can be used for tumor treatment or as active ingredients for preparing drugs for tumor treatment. The tumors described herein may comprise any tumors known to be treated with IFNα as disclosed, including but not limited to those listed previously.

This disclosure further comprises methods for treating or preventing tumors, comprising administering to a subject in need a therapeutically or prophylactically effective amount of the conjugates or the pharmaceutical compositions as described herein. This method can be used in combination with any known radiotherapy or immunotherapy.

Therefore, the present disclosure constructs a cytokine prodrug, wherein the prodrug chemically couples the masker (R) to the mutated cytokine through a chemical linker, thereby losing the activity of the cytokine in normal tissues and reducing the toxicity of the cytokine. Additionally, the cytokine prodrug can be further conjugated with antibodies to enhance drug performance (e.g., further optimization of targeting, half-life, and expression). Near the target tissue, the prodrug is activated by enzymatic cleavage or acidic conditions, restoring the cytokine to its original or better activity to exert therapeutic effects.

### Combination of biomolecular conjugates with antibodies

The present disclosure provides a method of combination therapy, comprising a method of combining the biomolecular conjugate with an anti-tumor antibody.

The anti-tumor antibody may comprise, for example: anti-PD-1 antibody, anti-Her2 antibody, anti-EGFR antibody, anti-VEGFR antibody, anti-CD20 antibody, anti-CD33 antibody, anti-PD-L1 antibody, anti-CTLA-4 antibody, anti-TNFα. antibody, anti-CD28 antibody, anti-4-1BB antibody, anti-OX40 antibody, anti-GITR antibody, anti-CD27 antibody, anti-CD40 antibody or anti-ICOS antibody, anti-CD25 antibody, anti-CD30 antibody, anti-CD3 antibody, anti-CD22 antibody, anti- CCR4 antibody, anti-CD38 antibody, anti-CD52 antibody, anti-complement C5 antibody, anti-F protein of RSV, anti-GD2 antibody, anti-GITR antibody, anti-glycoprotein receptor lib/Illa antibody, anti-ICOS antibody, anti-IL2R antibody, anti-LAG3 antibody, anti-integrin α4 antibody, anti-lgE antibody, anti-PDGFRa antibody, anti-RANKL antibody, anti-SLAMF7 antibody, anti-LTIGIT antibody, anti-TIM-3 antibody, anti-VEGFR2 antibody, anti-VISTA antibody, or functional fragments thereof.

As a preferred embodiment of the present disclosure, the anti-tumor antibody is an anti-PD-1 antibody. In specific examples of the present disclosure, the combination of the anti-tumor antibody with the biomolecule conjugate of the present disclosure shows a very significant synergy.

The present disclosure provides a use of the biomolecular conjugate and an anti-tumor antibody (for example, an anti-PD-1 antibody) for preparing a tumor-inhibiting mixture, pharmaceutical composition or drug kit.

During administration, the biomolecular conjugate and the anti-tumor antibody can be administered separately or simultaneously. It is understood that various administration methods are contemplated by the present disclosure.

The anti-tumor antibody may be murine, human, humanized, chimeric or from other species. The antibody may be of any type (e.g., IgG, IgE, IgM, IgD and IgA), class (e.g., IgG1, IgG2, IgG3, IgG4, IgA1 and IgA2) or subclass of immunoglobulin molecule. The antibody can be from any species. As some embodiments, the antibody is derived from human, mouse or rabbit.

Antibody fragments or binding portions of the antibody may also be used in the present disclosure. The antibody fragment or binding portion of an antibody comprises a portion of the entire length of the antibody, typically the antigen-binding or variable region of the antibody. Preferably, the fragment of the antibody is a functional fragment, i.e., retaining antigen-binding ability of the intact antibody. Examples of antibody fragments or functional fragments comprise Fab, Fab', F(ab')2 and Fv fragments; diabodies; linear antibodies; and single chain antibody molecules (scFv); and so on.

The present disclosure provides a mixture comprising the biomolecular conjugate and an anti-tumor antibody as active components. Preferably, in the mixture, the biomolecular conjugate and the tumor-targeted antibody are at a mass ratio of 1: (0.05-50), such as 1: 0.1, 1: 0.15, 1: 0.2, 1: 0.4, 1: 0.5, 1: 0.6, 1: 0.8, 1: 1, 1: 2, 1: 4, 1: 5, 1: 6, 1: 8, 1: 10, 1: 20, 1: 30, 1: 40.

The present disclosure provides a pharmaceutical composition comprising: (a) an effective amount of the biomolecular conjugate; (b) an effective amount of an anti-tumor antibody; and (c) a pharmaceutically acceptable carrier or excipient. Preferably, in the composition, the biomolecular conjugate and the tumor-targeted antibody are at a mass ratio of 1: (0.05-50), such as 1: 0.1, 1: 0.15, 1: 0.2, 1: 0.4, 1: 0.5, 1: 0.6, 1: 0.8, 1: 1, 1: 2, 1: 4, 1: 5, 1: 6, 1: 8, 1: 10, 1: 20, 1: 30, 1: 40.

The pharmaceutical composition or mixture of the present disclosure can be prepared into any conventional preparation form by conventional methods. Dosage forms can be diverse, the dosage form is acceptable as long as the active ingredients can effectively reach the body of the mammal. For example, it can be selected from: injection, infusion, tablet, capsule, pill. The active ingredient may be present in a suitable solid or liquid carrier or diluent. Suitable pharmaceutically acceptable carriers are the same as mentioned above.

The mixture or pharmaceutical composition of the biomolecular conjugate and the tumor-targeted antibody of the present disclosure may also be stored in a sterile device suitable for injection or infusion. Usually, in the pharmaceutical composition of the present disclosure, biomolecular conjugate and the tumor-targeted antibody, acted as active ingredients, can account for 0.0001-20% of the total weight of the pharmaceutical composition, and the rest can be a pharmaceutically acceptable carrier.

The effective amount of the biomolecular conjugate and the tumor-targeted antibody may vary with the route of administration and the severity of the disease to be treated. When necessary, the biomolecular conjugate can also be administered in combination with other active ingredients or drugs.

When the biomolecular conjugate used in combination with an anti-tumor antibody, the present disclosure also provides a drug kit for treating tumors. The kit comprises: a container 1 and the biomolecular conjugate placed in the container 1; and a container 2 and the anti-tumor antibody placed in the container 2.

The drug kit may also comprise a mixture of the biomolecular conjugate and the anti-tumor antibody, wherein the contents of the biomolecular conjugate and the anti-tumor antibody are as described above.

In addition, there may also be some auxiliary materials for medication in the drug kit, such as syringes for injections and so on.

In addition, the drug kit can also comprise instructions for use, illustrating the method of inhibiting tumors by using the method of combination in the present disclosure.

The present disclosure discloses for the first time that the combination of the biomolecular conjugate and the anti-tumor antibody has excellent anti-tumor effects and can achieve extremely significant synergistic effects. This effects greatly exceeds the expectations of ordinary people/physicians in this field.

The disclosure is further described below in conjunction with specific embodiments. It should be understood that these examples are merely for illustrative purposes rather than intended to limit the scope of the disclosure. The experimental methods without specifying the specific conditions in the following examples generally used the conventional conditions, such as those described in J. Sambrook, Molecular Cloning: A Laboratory Manual (3^{rd} ed. Science Press) or followed the manufacturer's recommendation.

The amino acid sequence of INF-alpha(SEQ ID NO: 1):











The biomolecular conjugate formed by cytokine IFNα has a structure as follows:

**R1-AAN-PABC-R3-S-Cys-IFNα**

wherein,
Cys represents a cysteine residue contained in IFNα;
S represents a sulfur atom on the cysteine residue;
R1 is a group that prevents IFNα proteins from binding to its ligand or receptor;
AAN represents a tripeptide, wherein A is alanine, N is aspartic acid;
PABC is para-aminobenzyl carbamate;
R3 is a group comprising an EMC group that can covalently conjugate to the sulfur atom in the cysteine residue. The function of R3 is to maintain or improve the binding ability of IFNα to its antigen after AAN is cleaved.

In subsequent examples, R1 used is as shown in Table 1.

**Table 1**

| Name | Molecular formula |
|---|---|
| R1-12 | Peg-5000 |
| R1-13 | Peg-10000 |
| R1-15 | Peg-20000 |
| R1-17 | Peg-40000 |

In the experiments of the present disclosure, several linkers of **R1-AAN-PABC-R3** were selected for study. The synthesized linker compounds were uniformly dissolved and diluted tenfold with water to a concentration of 1 mg/mL. At 37°C for 2 hours, 1 mg/mL of the sample compound was added to 100 µg of legumain. The enzyme cleaves the linker, resulting in the release of R3. The reduction of the linker and the increase in R3 were analyzed using HPLC to compare the activation efficiency of the linker by the enzyme (defined as the proportion of R3 released from the original compound through enzymatic cleavage). As shown in Table 2, when R1 = peg5000, the linkers and their activation efficiencies are as shown in Table 2.

**Table 2**

| Name | Linker | Activation efficiency (%) | pH 7.4 |
|---|---|---|---|
| 1 | 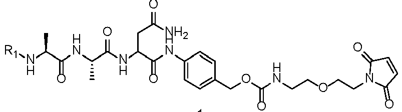 | 88.6 | Stable |
| 2 | 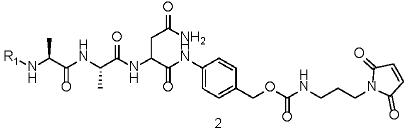 | 60.1 | Stable |
| 3 | 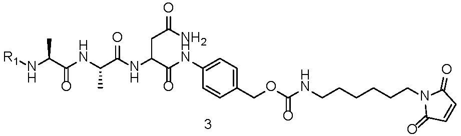 | 91.5 | Stable |
| 4 | 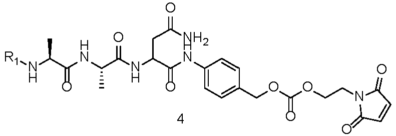 | 75.7 | Stable |
| 5 | 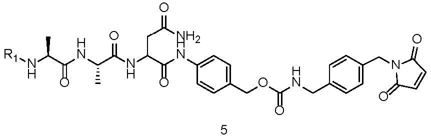 | 54.6 | Stable |
| 6 | 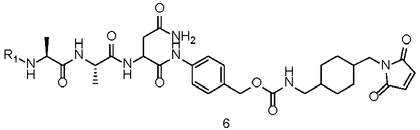 | 64.3 | Stable |
| 7 | 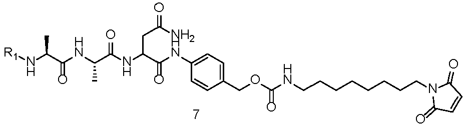 | 75.6 | Stable |

When R1 = peg20000, preferable linker R1-AAN-PABC-R3 and its activation efficiency are as shown in Table 3.

**Table 3**

| Name | Linker | Activation efficiency (%) | pH 7.4 |
|---|---|---|---|
| 8 | 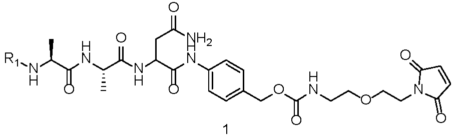 | 75.3 | Stable |
| 9 | 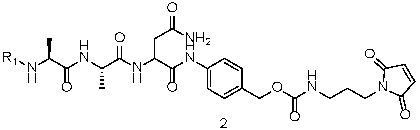 | 50.5 | Stable |
| 10 | 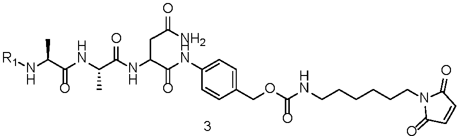 | 86.7 | Stable |
| 11 | 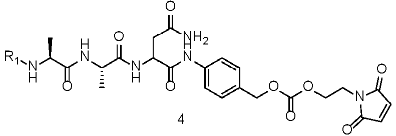 | 65.2 | Stable |
| 12 | 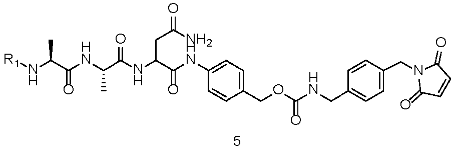 | 30.8 | Stable |
| 13 | 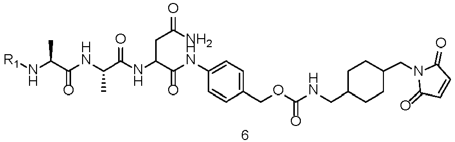 | 45.9 | Stable |
| 14 | 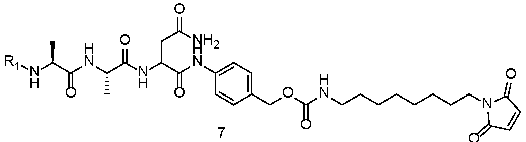 | 55.6 | Stable |

The above results indicate that, based on a comprehensive evaluation of stability and activation efficiency, both linker 1 and linker 3 exhibit very high activation efficiency and linker 10 also demonstrates a high activation efficiency.

### Example 1. Conjugation of IFN-α proteins with amino acid mutations on the surface and the R1-AAN-PABC-R3 compound.

The linker **R1-AAN-PABC-R3,** that is linker 3 from Table 2, was used, wherein R1 is peg5000.

IFN-α protein with any amino acid mutation to Cys on the surface can be conjugated with the **R1-AAN-PABC-R3** compound, but conjugation efficiency varies. IFN-α protein was mutated at positions 2-165 of the amino acid sequence by single-point mutation. Based on mutated IFN-α amino acid sequences and DNA sequences thereof, plasmids capable of expressing His*6-INF-α in mammalian cells were constructed (GENEWIZ). The expression host was HEK293 cells (Life Technologies). Prior to transfection, HEK293 cells were cultured in complete medium comprising 10% FBS (Gibco) at 37°C with 5% CO₂. On the day before transfection, cells were seeded at an appropriate density in 15 cm culture dishes and the medium was replaced with low IgG FBS. Six hours or two days after transfection, the medium was replaced with Freestyle293 medium (Gibco). On the day of transfection, when cells reached a certain confluency, plasmids encoding the target protein were co-transfected into 293T cells using Lipofectamine 2000 (Life Technologies) and PEI (Sigma). Supernatants were collected on days 4 and 6 post-transfection to detect protein expression and activity, followed by protein purification.

The purified mutants were incubated at a concentration of 0.3 mg/mL in 20 mM phosphate buffer (pH 7.4) comprising 5 mM EDTA. TCEP solution was added to the mixture at a 100:1 molar ratio and the mixture was incubated at 4°C for 4 hours with gentle stirring. The mixture was then dialyzed against 20 mM phosphate buffer (pH 7.4) containing 150 mM NaCl at 4°C for 2 hours. Immediately after dialysis, **R1-AAN-PABC-R3** was added to the mixture at a 20:1 molar ratio and the mixture was gently stirred at 25°C for 16 hours. The reaction was stopped and residual **R1-AAN-PABC-R3** compounds were removed. Equal volumes of samples were subjected to SDS-PAGE. Conjugation efficiency was determined by scanning the grayscale intensity of the protein bands (the ratio of the scanned grayscale is the conjugation efficiency: grayscale of conjugated protein band/ (grayscale of unbounded protein band + grayscale of conjugated protein band)). Conjugation efficiencies of IFN-α proteins with single-point Cys mutations with the **R1-AAN-PABC-R3** compound are listed in Table 4.

**Table 4**

| Amino acid | Position | Conjugation Efficiency |
|---|---|---|
| Asp | 2 | 30-50% |
| Leu | 3 | 30-50% |
| Pro | 4 | 80-90% |
| Gln | 5 | 80-90% |
| Thr | 6 | 80-90% |
| His | 7 | 80-90% |
| Ser | 8 | 30-50% |
| Leu | 9 | 80-90% |
| Gly | 10 | 30-50% |
| Arg | 12 | 80-90% |
| Arg | 13 | 60-70% |
| Leu | 15 | 30-50% |
| Met | 16 | 80-90% |
| Leu | 17 | 30-50% |
| Ala | 19 | 30-50% |
| Gln | 20 | 80-90% |
| Arg | 22 | 30-50% |
| Lys | 23 | 60-70% |
| Ile | 24 | 60-70% |
| Ser | 25 | 80-90% |
| Leu | 26 | 30-50% |
| Phe | 27 | 80-90% |
| Ser | 28 | 80-90% |
| Leu | 30 | 80-90% |
| Lys | 31 | 80-90% |
| Asp | 32 | 30-50% |
| Arg | 33 | 80-90% |
| His | 34 | 80-90% |
| Asp | 35 | 60-70% |
| Phe | 36 | 60-70% |
| Gly | 37 | 80-90% |
| Phe | 38 | 30-50% |
| Pro | 39 | 30-50% |
| Gln | 40 | 60-70% |
| Glu | 41 | 80-90% |
| Glu | 42 | 60-70% |
| Gly | 44 | 60-70% |
| Asn | 45 | 80-90% |
| Gln | 46 | 60-70% |
| Phe | 47 | 30-50% |
| Gln | 48 | 60-70% |
| Lys | 49 | 80-90% |
| Ala | 50 | 30-50% |
| Glu | 51 | 80-90% |
| Pro | 54 | 30-50% |
| His | 57 | 60-70% |
| Glu | 58 | 80-90% |
| Gln | 61 | 60-70% |
| Gln | 62 | 30-50% |
| Phe | 64 | 80-90% |
| Asn | 65 | 80-90% |
| Leu | 66 | 30-50% |
| Ser | 68 | 60-70% |
| Thr | 69 | 60-70% |
| Lys | 70 | 80-90% |
| Asp | 71 | 30-50% |
| Ser | 73 | 60-70% |
| Ala | 74 | 80-90% |
| Asp | 77 | 60-70% |
| Glu | 78 | 80-90% |
| Thr | 79 | 80-90% |
| Leu | 80 | 30-50% |
| Asp | 82 | 80-90% |
| Lys | 83 | 60-70% |
| Tyr | 85 | 60-70% |
| Thr | 86 | 80-90% |
| Glu | 87 | 30-50% |
| Tyr | 89 | 80-90% |
| Gln | 90 | 60-70% |
| Leu | 92 | 30-50% |
| Asn | 93 | 80-90% |
| Asp | 94 | 30-50% |
| Glu | 96 | 60-70% |
| Ala | 97 | 60-70% |
| Val | 99 | 60-70% |
| Ile | 100 | 80-90% |
| Gln | 101 | 80-90% |
| Gly | 102 | 60-70% |
| Val | 103 | 60-70% |
| Gly | 104 | 80-90% |
| Val | 105 | 30-50% |
| Glu | 107 | 80-90% |
| Thr | 108 | 60-70% |
| Pro | 109 | 60-70% |
| Leu | 110 | 80-90% |
| Met | 111 | 30-50% |
| Lys | 112 | 60-70% |
| Glu | 113 | 80-90% |
| Asp | 114 | 60-70% |
| Leu | 117 | 80-90% |
| Ala | 118 | 60-70% |
| Arg | 120 | 30-50% |
| Lys | 121 | 80-90% |
| Tyr | 122 | 60-70% |
| Gln | 124 | 60-70% |
| Arg | 125 | 80-90% |
| Thr | 127 | 30-50% |
| Leu | 128 | 80-90% |
| Tyr | 129 | 60-70% |
| Lys | 131 | 80-90% |
| Glu | 132 | 80-90% |
| Lys | 133 | 60-70% |
| Lys | 134 | 80-90% |
| Tyr | 135 | 60-70% |
| Ser | 136 | 60-70% |
| Pro | 137 | 80-90% |
| Glu | 141 | 60-70% |
| Val | 142 | 60-70% |
| Arg | 144 | 30-50% |
| Ala | 145 | 80-90% |
| Glu | 146 | 60-70% |
| Met | 148 | 60-70% |
| Arg | 149 | 80-90% |
| Ser | 152 | 80-90% |
| Leu | 153 | 60-70% |
| Thr | 155 | 30-50% |
| Asn | 156 | 80-90% |
| Leu | 157 | 60-70% |
| Gln | 158 | 60-70% |
| Glu | 159 | 30-50% |
| Ser | 160 | 60-70% |
| Leu | 161 | 80-90% |
| Arg | 162 | 80-90% |
| Ser | 163 | 80-90% |
| Lys | 164 | 80-90% |
| Glu | 165 | 80-90% |

### Example 2. Receptor binding ability after conjugation of IFN-α proteins with amino acid mutations on the surface and the R1-AAN-PABC-R3 compound.

IFN-α protein was mutated at positions 2-165 of the amino acid sequence by single-point mutation. The binding activity of each mutant after conjugation compared to that of wild-type IFN-α was detected by ELISA. Specifically, a 96-well plate was coated overnight with INFR1 or INFR2 receptors, then blocked with 1% BSA blocking solution (ThermoFisher) at 37°C for 2 hours and washed three times with PBST. Corresponding antibodies or mutants were added to the wells and incubated at 37°C for 1 hour, followed by washing three times with PBST. HRP-conjugated anti-human IFN-α 2b antibodies were added and incubated at 37°C for 1 hour, followed by washing three times with PBST. TMB substrate (Solarbio, Inc.) was added and absorbance was measured at 450 nm. The effect on binding strength of conjugated mutants were calculated by EC50 after conjugation/wild type EC50, that is (binding ability after conjugation/binding ability of wild type) *100%=1/ (EC50 after conjugation/wild-type EC50).

Receptor binding abilities after conjugation of IFN-α proteins with single-point Cys mutation with the **R1-AAN-PABC-R3** compound are listed in Table 5. The linker **R1-AAN-PABC-R3** is linker 3 from Table 2, wherein R1 is peg5000.

**Table 5**

| Amino acid | Position | (binding ability after conjugation/binding ability of wild type) *100% |
|---|---|---|
| Asp | 2 | >80% |
| Leu | 3 | >80% |
| Pro | 4 | >80% |
| Gln | 5 | >80% |
| Thr | 6 | >80% |
| His | 7 | >80% |
| Ser | 8 | >80% |
| Leu | 9 | >80% |
| Gly | 10 | >80% |
| Arg | 12 | 10-30% |
| Arg | 13 | 50-80% |
| Leu | 15 | 30-50% |
| Met | 16 | <10% |
| Leu | 17 | 50-80% |
| Ala | 19 | 10-30% |
| Gln | 20 | 10-30% |
| Arg | 22 | 10-30% |
| Lys | 23 | 30-50% |
| Ile | 24 | 50-80% |
| Ser | 25 | <10% |
| Leu | 26 | 30-50% |
| Phe | 27 | <10% |
| Ser | 28 | 10-30% |
| Leu | 30 | <10% |
| Lys | 31 | 10-30% |
| Asp | 32 | 30-50% |
| Arg | 33 | <10% |
| His | 34 | 10-30% |
| Asp | 35 | 50-80% |
| Phe | 36 | >80% |
| Gly | 37 | 50-80% |
| Phe | 38 | 30-50% |
| Pro | 39 | >80% |
| Gln | 40 | 30-50% |
| Glu | 41 | 30-50% |
| Glu | 42 | >80% |
| Gly | 44 | 50-80% |
| Asn | 45 | 50-80% |
| Gln | 46 | >80% |
| Phe | 47 | >80% |
| Gln | 48 | >80% |
| Lys | 49 | >80% |
| Ala | 50 | >80% |
| Glu | 51 | >80% |
| Pro | 54 | >80% |
| His | 57 | 10-30% |
| Glu | 58 | 10-30% |
| Gln | 61 | 10-30% |
| Gln | 62 | 30-50% |
| Phe | 64 | 30-50% |
| Asn | 65 | 10-30% |
| Leu | 66 | 50-80% |
| Ser | 68 | 30-50% |
| Thr | 69 | 10-30% |
| Lys | 70 | 30-50% |
| Asp | 71 | >80% |
| Ser | 73 | 30-50% |
| Ala | 74 | >80% |
| Asp | 77 | >80% |
| Glu | 78 | >80% |
| Thr | 79 | 50-80% |
| Leu | 80 | >80% |
| Asp | 82 | 50-80% |
| Lys | 83 | 50-80% |
| Tyr | 85 | 30-50% |
| Thr | 86 | 10-30% |
| Glu | 87 | 50-80% |
| Tyr | 89 | 10-30% |
| Gln | 90 | 30-50% |
| Leu | 92 | >80% |
| Asn | 93 | 10-30% |
| Asp | 94 | 50-80% |
| Glu | 96 | 30-50% |
| Ala | 97 | >80% |
| Val | 99 | >80% |
| Ile | 100 | >80% |
| Gln | 101 | >80% |
| Gly | 102 | >80% |
| Val | 103 | >80% |
| Gly | 104 | >80% |
| Val | 105 | >80% |
| Glu | 107 | >80% |
| Thr | 108 | >80% |
| Pro | 109 | >80% |
| Leu | 110 | >80% |
| Met | 111 | >80% |
| Lys | 112 | >80% |
| Glu | 113 | >80% |
| Asp | 114 | >80% |
| Leu | 117 | 30-50% |
| Ala | 118 | >80% |
| Arg | 120 | 30-50% |
| Lys | 121 | 30-50% |
| Tyr | 122 | >80% |
| Gln | 124 | 10-30% |
| Arg | 125 | 50-80% |
| Thr | 127 | 30-50% |
| Leu | 128 | 50-80% |
| Tyr | 129 | >80% |
| Lys | 131 | 30-50% |
| Glu | 132 | 50-80% |
| Lys | 133 | >80% |
| Lys | 134 | >80% |
| Tyr | 135 | 30-50% |
| Ser | 136 | >80% |
| Pro | 137 | >80% |
| Glu | 141 | 10-30% |
| Val | 142 | <10% |
| Arg | 144 | 10-30% |
| Ala | 145 | <10% |
| Glu | 146 | 30-50% |
| Met | 148 | 10-30% |
| Arg | 149 | <10% |
| Ser | 152 | <10% |
| Leu | 153 | 10-30% |
| Thr | 155 | 30-50% |
| Asn | 156 | <10% |
| Leu | 157 | 10-30% |
| Gln | 158 | 10-30% |
| Glu | 159 | 30-50% |
| Ser | 160 | 10-30% |
| Leu | 161 | 10-30% |
| Arg | 162 | 10-30% |
| Ser | 163 | 10-30% |
| Lys | 164 | 30-50% |
| Glu | 165 | 30-50% |

### Example 3. Receptor binding ability after conjugation and activation of IFN-α proteins with amino acid mutations on the surface and the R1-AAN-PABC-R3 compound.

IFN-α protein was mutated at positions 2-165 of the amino acid sequence by single-point mutation, followed by an activation assay. In the activation assay, the conjugated mutant at a concentration of 1 mg/mL was incubated overnight in an acidic environment (pH = 6) at 37°C. Receptor binding after activation was analyzed using ELISA.

Receptor binding abilities after conjugation and activation of IFN-α proteins with single-point Cys mutation with the **R1-AAN-PABC-R3** compound are listed in Table 6. The linker **R1-AAN-PABC-R3** is linker 3 from Table 2, wherein R1 is peg5000.

**Table 6**

| Amino acid | Position | (EC50 after conjugation and activation/wild-type EC50) *100% |
|---|---|---|
| Asp | 2 | 70-150% |
| Leu | 3 | 70-150% |
| Pro | 4 | 70-150% |
| Gln | 5 | 70-150% |
| Thr | 6 | 70-150% |
| His | 7 | 150-300% |
| Ser | 8 | 70-150% |
| Leu | 9 | 70-150% |
| Gly | 10 | 150-300% |
| Arg | 12 | >300% |
| Arg | 13 | 150-300% |
| Leu | 15 | 70-150% |
| Met | 16 | >300% |
| Leu | 17 | 150-300% |
| Ala | 19 | 70-150% |
| Gln | 20 | >300% |
| Arg | 22 | 70-150% |
| Lys | 23 | 150-300% |
| Ile | 24 | 70-150% |
| Ser | 25 | >300% |
| Leu | 26 | 150-300% |
| Phe | 27 | 150-300% |
| Ser | 28 | 150-300% |
| Leu | 30 | >300% |
| Lys | 31 | >300% |
| Asp | 32 | 150-300% |
| Arg | 33 | >300% |
| His | 34 | 150-300% |
| Asp | 35 | 150-300% |
| Phe | 36 | 70-150% |
| Gly | 37 | 70-150% |
| Phe | 38 | 150-300% |
| Pro | 39 | 70-150% |
| Gln | 40 | 70-150% |
| Glu | 41 | 150-300% |
| Glu | 42 | 70-150% |
| Gly | 44 | 70-150% |
| Asn | 45 | 70-150% |
| Gln | 46 | 70-150% |
| Phe | 47 | 70-150% |
| Gln | 48 | 70-150% |
| Lys | 49 | 70-150% |
| Ala | 50 | 70-150% |
| Glu | 51 | 70-150% |
| Pro | 54 | 70-150% |
| His | 57 | 70-150% |
| Glu | 58 | 150-300% |
| Gln | 61 | <70% |
| Gln | 62 | <70% |
| Phe | 64 | <70% |
| Asn | 65 | 70-150% |
| Leu | 66 | 70-150% |
| Ser | 68 | <70% |
| Thr | 69 | 70-150% |
| Lys | 70 | 70-150% |
| Asp | 71 | 70-150% |
| Ser | 73 | <70% |
| Ala | 74 | 70-150% |
| Asp | 77 | 70-150% |
| Glu | 78 | 70-150% |
| Thr | 79 | 70-150% |
| Leu | 80 | 70-150% |
| Asp | 82 | 70-150% |
| Lys | 83 | 70-150% |
| Tyr | 85 | 70-150% |
| Thr | 86 | 70-150% |
| Glu | 87 | 70-150% |
| Tyr | 89 | 70-150% |
| Gln | 90 | 150-300% |
| Leu | 92 | 70-150% |
| Asn | 93 | 70-150% |
| Asp | 94 | 70-150% |
| Glu | 96 | 70-150% |
| Ala | 97 | 70-150% |
| Val | 99 | 70-150% |
| Ile | 100 | 70-150% |
| Gln | 101 | 70-150% |
| Gly | 102 | 70-150% |
| Val | 103 | 70-150% |
| Gly | 104 | 70-150% |
| Val | 105 | 70-150% |
| Glu | 107 | 70-150% |
| Thr | 108 | 70-150% |
| Pro | 109 | 70-150% |
| Leu | 110 | 70-150% |
| Met | 111 | 70-150% |
| Lys | 112 | 70-150% |
| Glu | 113 | 70-150% |
| Asp | 114 | 70-150% |
| Leu | 117 | 70-150% |
| Ala | 118 | 70-150% |
| Arg | 120 | 70-150% |
| Lys | 121 | 70-150% |
| Tyr | 122 | 70-150% |
| Gln | 124 | 70-150% |
| Arg | 125 | 70-150% |
| Thr | 127 | 70-150% |
| Leu | 128 | 70-150% |
| Tyr | 129 | 70-150% |
| Lys | 131 | 150-300% |
| Glu | 132 | 70-150% |
| Lys | 133 | 70-150% |
| Lys | 134 | 70-150% |
| Tyr | 135 | 70-150% |
| Ser | 136 | 70-150% |
| Pro | 137 | 70-150% |
| Glu | 141 | >300% |
| Val | 142 | 150-300% |
| Arg | 144 | >300% |
| Ala | 145 | 150-300% |
| Glu | 146 | 150-300% |
| Met | 148 | >300% |
| Arg | 149 | 150-300% |
| Ser | 152 | 150-300% |
| Leu | 153 | 150-300% |
| Thr | 155 | 150-300% |
| Asn | 156 | >300% |
| Leu | 157 | 150-300% |
| Gln | 158 | >300% |
| Glu | 159 | 150-300% |
| Ser | 160 | 150-300% |
| Leu | 161 | >300% |
| Arg | 162 | 70-150% |
| Ser | 163 | 150-300% |
| Lys | 164 | 70-150% |
| Glu | 165 | 70-150% |

### Example 4. Binding activity after conjugation of mutated IFN-α with different R1-AAN-PABC-R3 compounds.

IFN-α protein was mutated at positions 2-165 of the amino acid sequence by single-point mutation. Based on INF-α and mutated IFN-α amino acid sequences and DNA sequences thereof, plasmids capable of expressing His*6-INF-α in mammalian cells were constructed (GENEWIZ). The expression host was HEK293 cells (Life Technologies). Prior to transfection, HEK293 cells were cultured in complete medium comprising 10% FBS (Gibco) at 37°C with 5% CO₂. On the day before transfection, cells were seeded at an appropriate density in 15 cm culture dishes and the medium was replaced with low IgG FBS. Six hours or two days after transfection, the medium was replaced with Freestyle293 medium (Gibco). On the day of transfection, when cells reached a certain confluency, plasmids encoding the target protein were co-transfected into 293T cells using Lipofectamine 2000 (Life Technologies) and PEI (Sigma). Supernatants were collected on days 4 and 6 post-transfection to detect protein expression and activity, followed by protein purification.

The purified mutants were incubated at a concentration of 0.3 mg/mL in 20 mM phosphate buffer (pH 7.4) comprising 5 mM EDTA. TCEP solution was added to the mutants at a 100:1 molar ratio and the mixture was incubated at 4°C for 4 hours with gentle stirring. The mixture was then dialyzed against 20 mM phosphate buffer (pH 7.4) containing 150 mM NaCl at 4°C for 2 hours. Immediately after dialysis, **R1-AAN-PABC-R3** was added to the mixture at a 20:1 molar ratio and the mixture was gently stirred at 25°C for 16 hours. The reaction was stopped and residual **R1-AAN-PABC-R3** compounds were removed.

In the present disclosure, the binding activity after conjugation of each mutant with single-point mutation compared to wild-type INF-α was tested by ELISA. Specifically, a 96-well plate was coated overnight with INFR1 or INFR2 receptors, then blocked with 1% BSA blocking solution (ThermoFisher) at 37°C for 2 hours and washed three times with PBST. Corresponding antibodies or mutants were added to the wells and incubated at 37°C for 1 hour, followed by washing three times with PBST. HRP-conjugated anti-human IFN-α 2b antibodies were added and incubated at 37°C for 1 hour, followed by washing three times with PBST. TMB substrate (Solarbio, Inc.) was added and absorbance was measured at 450 nm. The effect on binding strength of conjugated mutants were calculated by EC50 after conjugation/wild type EC50, that is (binding ability after conjugation/binding ability of wild type)*100%=1/(EC50 after conjugation/wild-type EC50).

According to the above method, binding activity after conjugation of mutated IFN-α with different **R1-AAN-PABC-R3** compounds was evaluated.

Binding activity after conjugation of mutated IFN-α with R was as shown in Table 7.

**Table 7**

| Amino acid | Position | (AAN-PABC-R3) | R1 | inhibitory ligand | (binding ability after conjugation/binding ability of wild type) *100% |
|---|---|---|---|---|---|
| His | 57 | 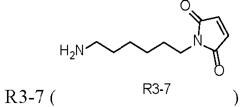 | R1-12 | INFR1 | 25.1% |
| His | 57 | R3-7 | R1-13 | INFR1 | 22.6% |
| His | 57 | R3-7 | R1-15 | INFR1 | 4.6% |
| His | 57 | R3-7 | R1-17 | INFR1 | 3.7% |
| Gln | 61 | R3-7 | R1-12 | INFR1 | 23.6% |
| Gln | 61 | R3-7 | R1-13 | INFR1 | 5.3% |
| Gln | 61 | R3-7 | R1-15 | INFR1 | 2.8% |
| Gln | 61 | R3-7 | R1-17 | INFR1 | 0.78% |
| Phe | 64 | R3-7 | R1-12 | INFR1 | 34.2% |
| Phe | 64 | R3-7 | R1-13 | INFR1 | 19.9% |
| Phe | 64 | R3-7 | R1-15 | INFR1 | 7.8% |
| Phe | 64 | R3-7 | R1-17 | INFR1 | 4.8% |
| Ser | 68 | R3-7 | R1-12 | INFR1 | 27.7% |
| Ser | 68 | R3-7 | R1-13 | INFR1 | 21.4% |
| Ser | 68 | R3-7 | R1-15 | INFR1 | 4.7% |
| Ser | 68 | R3-7 | R1-17 | INFR1 | 2.6% |
| Phe | 27 | R3-7 | R1-12 | INFR2 | 5.7% |
| Phe | 27 | R3-7 | R1-13 | INFR2 | 1.68% |
| Phe | 27 | R3-7 | R1-15 | INFR2 | 0.59% |
| Phe | 27 | R3-7 | R1-17 | INFR2 | 0.12% |
| Ala | 145 | R3-7 | R1-12 | INFR2 | 3.1% |
| Ala | 145 | R3-7 | R1-13 | INFR2 | 0.21% |
| Ala | 145 | R3-7 | R1-15 | INFR2 | 0.01% |
| Ala | 145 | R3-7 | R1-17 | INFR2 | 0.01% |
| Ser | 152 | R3-7 | R1-12 | INFR2 | 8.4% |
| Ser | 152 | R3-7 | R1-13 | INFR2 | 6.5% |
| Ser | 152 | R3-7 | R1-15 | INFR2 | 2.3% |
| Ser | 152 | R3-7 | R1-17 | INFR2 | 1.2% |
| Arg | 149 | R3-7 | R1-12 | INFR2 | 4.3% |
| Arg | 149 | R3-7 | R1-13 | INFR2 | 1.0% |
| Arg | 149 | R3-7 | R1-15 | INFR2 | 0.27% |
| Arg | 149 | R3-7 | R1-17 | INFR2 | 0.07% |
| Arg | 162 | R3-7 | R1-12 | INFR2 | 20.4% |
| Arg | 162 | R3-7 | R1-13 | INFR2 | 12.8% |
| Arg | 162 | R3-7 | R1-15 | INFR2 | 5.1% |
| Arg | 162 | R3-7 | R1-17 | INFR2 | 4.9% |

Therefore, for mutant positions that inhibit INFR1 binding, after His57, Gln61, Phe64 or Ser68 was mutated to Cys respectively, and then conjugated with certain **R1-AAN-PABC-R3,** its ability to bind to INFR1 was reduced to below 10% of the wild type. For mutant positions that inhibit INFR2 binding, after Ala145 or Arg149 was mutated to Cys respectively, and then conjugated with certain **R1-AAN-PABC-R3,** its ability to bind to INFR2 was reduced to below 1%, even below 0.1% of the wild type. Phe27, Ser152 or Arg162 also declined to varying degrees.

### Example 5. Binding activity after conjugation and activation of mutated IFN-α with R1-AAN-PABC-R3.

In the activation assay, 10 µg of activation-specific Legumain enzyme (capable of cleaving at the position of AAN) was added to 1 mg/mL of the conjugated mutant and incubated at 37°C for 1 hour. Alternatively, 1 mg/mL of the conjugated mutant was placed in a pH=6 acidic environment and incubated at 37°C for 4 hours. Receptor binding after activation was analyzed using ELISA.

Binding activity after conjugation and activation of mutated IFN-α with **R1-AAN-PABC-R3** was as shown in Table 8.

**Table 8**

| Amino acid | Position | (AAN-PABC-R3) | R1 | inhibitory ligand | (EC50 after activation/ wild-type EC50) |
|---|---|---|---|---|---|
| His | 57 | R3-7 | R1-15 | INFR1 | 1.2 |
| His | 57 | R3-7 | R1-15 | INFR1 | 0.32 |
| His | 57 | R3-7 | R1-15 | INFR1 | 2.4 |
| Gln | 61 | R3-7 | R1-15 | INFR1 | 0.56 |
| Gln | 61 | R3-7 | R1-15 | INFR1 | 0.48 |
| Gln | 61 | R3-7 | R1-15 | INFR1 | 1.1 |
| Phe | 64 | R3-7 | R1-15 | INFR1 | 0.42 |
| Phe | 64 | R3-7 | R1-15 | INFR1 | 0.30 |
| Phe | 64 | R3-7 | R1-15 | INFR1 | 0.51 |
| Ser | 68 | R3-7 | R1-15 | INFR1 | 0.45 |
| Ser | 68 | R3-7 | R1-15 | INFR1 | 0.30 |
| Ser | 68 | R3-7 | R1-15 | INFR1 | 0.67 |
| Phe | 27 | R3-7 | R1-15 | INFR2 | 2.6 |
| Phe | 27 | R3-7 | R1-15 | INFR2 | 1.2 |
| Phe | 27 | R3-7 | R1-15 | INFR2 | 1.9 |
| Ala | 145 | R3-7 | R1-15 | INFR2 | 2.2 |
| Ala | 145 | R3-7 | R1-15 | INFR2 | 1.6 |
| Ala | 145 | R3-7 | R1-15 | INFR2 | 4.1 |
| Ser | 152 | R3-7 | R1-15 | INFR2 | 1.7 |
| Ser | 152 | R3-7 | R1-15 | INFR2 | 1.8 |
| Ser | 152 | R3-7 | R1-15 | INFR2 | 1.9 |
| Arg | 149 | R3-7 | R1-15 | INFR2 | 1.9 |
| Arg | 149 | R3-7 | R1-15 | INFR2 | 0.96 |
| Arg | 149 | R3-7 | R1-15 | INFR2 | 1.2 |
| Arg | 162 | R3-7 | R1-15 | INFR2 | 0.7 |
| Arg | 162 | R3-7 | R1-15 | INFR2 | 0.87 |
| Arg | 162 | R3-7 | R1-15 | INFR2 | 2.2 |

The results indicate that, for mutant positions aimed at inhibiting INFR1 binding, except for Phe64 and Ser68 with similar side chains to R3 structure, mutant corresponding to His57 with distinct structural difference to R3 structure and mutant corresponding to Gln61 with charged side chain, exhibit binding activity to INFR1 that is restored to more than 200% of the wild-type level after conjugation and activation. Since IFN-α has inherently high binding activity to INFR2, any modifications to IFN-α may result in reduced INFR2 binding. Therefore, the key to selecting mutant positions for INFR2 lies in the recovery of binding activity after conjugation and activation. For mutant positions designed to inhibit INFR2 binding, except for Phe27, Ala145 and Ser152 with similar side chains to R3 structure, mutant corresponding to Arg149 (R149, that is, Arg at position 149 is mutated to Cys) exhibit a declined binding activity to 0.27% of the original after conjugation, but its binding ability to INFR2 can be restored to nearly 100% of the wild-type level after activation.

### Example 6. Effects of conjugates of mutant IFN-α and R1-AAN-PABC-R3 on cellular signaling after conjugation and activation.

280,000 cells/ml HEK-Blue IFN-a/b cell suspension was prepared in culture medium containing 10% heat-inactivated FBS. 20 µL of different concentrations of IFN-α, mutant IFN-α + **R1-AAN-PABC-R3** (wherein R1 is peg20000), Active form were added into a 96-well cell plate, separately. Then 180 µl of cell suspension was added to each well. The 96-well cell plate was placed in a carbon dioxide incubator at 37°C for 20-24 hours.

QUANTI-Blue^{™} was prepared according to product instructions and 180 µl QUANTI-Blue^{™} was added to each well of a new 96-well cell plate. Then 20 µl of the above HEK-Blue IFN-a/b cell supernatant was added and incubate at 37°C for 1 hour. SEAP levels were measured at 620-055nm wavelength.

Effects of conjugates of mutant IFN-α and **R1-AAN-PABC-R3** on cellular signal transduction after conjugation and activation were shown in Table 9.

**Table 9**

| Amino acid | Position | (AAN-PABC-R3) | R1 | Effects on cellular signal transduction after conjugation | Effects on cellular signal transduction after activation |
|---|---|---|---|---|---|
| His | 57 | R3-7 | Peg20000 | 0.1% | 30% |
| Gln | 61 | R3-7 | Peg20000 | Signal transduction was not detected | 2% |
| Ser | 68 | R3-7 | Peg20000 | Signal transduction was not detected | 10% |
| Phe | 27 | R3-7 | Peg20000 | 0.05% | 23% |
| Ala | 145 | R3-7 | Peg20000 | Signal transduction was not detected | 36% |
| Arg | 149 | R3-7 | Peg20000 | Signal transduction was not detected | 90% |

| | | | | | |
|---|---|---|---|---|---|
| * Conjugated signaling/wild-type signaling* 100%. | | | | | |

The results of Table 6 indicate that the sample of INF-α R149 conjugated with **R1-AAN-PABC-R3** and its activated form exhibit the most optimal effect on cell signal transduction.

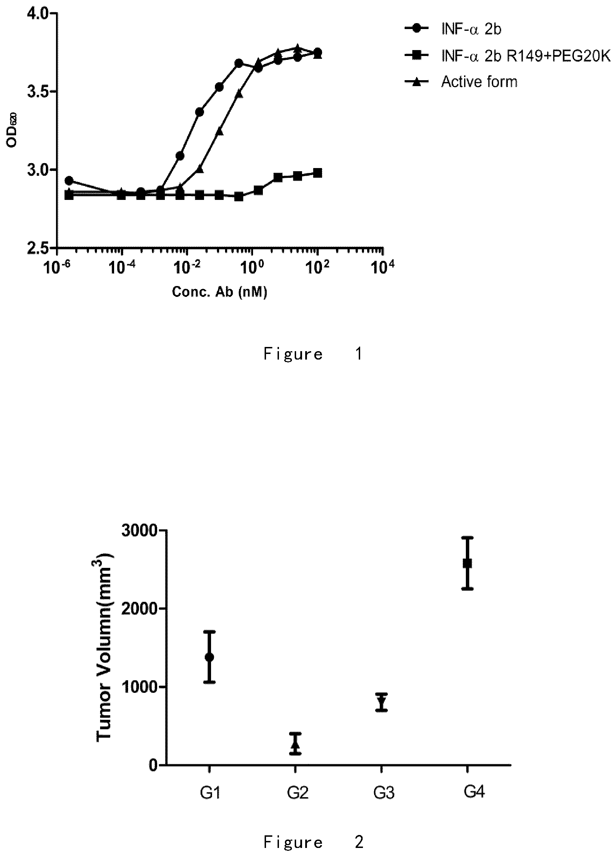

The effect of INF-α R149 conjugation and activation on cell signal transduction is shown in Figure 1. The results demonstrate that various mutant forms of INF-α conjugated with **R1-AAN-PABC-R3** significantly reduce or completely inhibit cell signal transduction. However, a remarkable restoration of signal transduction is observed after activation. Among them, the sample of INF-α R149 conjugated with **R1-AAN-PABC-R3** can entirely suppress the corresponding cell signal transduction, while the activated form restores cell signal transduction to a level equivalent to that of wild-type INF-α.

### Example 7. Growth inhibition of activated IFN-α in a tumor microenvironment on MC38 tumors

MC38 cells were subcutaneously implanted into C57BL/6 mice (provided by Shanghai Research Center of model organisms), with 2 × 10⁶ cells per mouse. After one week, mice implanted with MC38 tumors were randomly divided into groups. Group 1 (G1) was injected with a solvent control once weekly, while the other groups was injected with 100ug of mutant INF-α+**R1-AAN-PABC-R3,** administered once a week. The treatment cycle lasted for 3 weeks. Tumor volumes of the mice were recorded three times weekly. Tumor Growth Inhibition (TGI) effects of different INF-α + **R1-AAN-PABC-R3** treatments are shown in Table 10.

**Table 10**

| Amino acid | Position | (AAN-PABC-R3) | R1 | TGI (relative to Group G1 solvent control group, %) |
|---|---|---|---|---|
| His | 57 | R3-7 | Peg20000 | 26.3% |
| Gln | 61 | R3-7 | Peg20000 | No significance compared to the control |
| Phe | 64 | R3-7 | Peg20000 | 35.7% |
| Ser | 68 | R3-7 | Peg20000 | 5.1% |
| Phe | 27 | R3-7 | Peg20000 | 27.4% |
| Ser | 152 | R3-7 | Peg20000 | 37.5% |
| Ala | 145 | R3-7 | Peg20000 | 38.4% |
| Arg | 162 | R3-7 | Peg20000 | 30.1% |
| Arg | 149 | R3-7 | Peg220000 | 52.8% |

The results indicate that various mutant forms of INF-α conjugated with **R1-AAN-PABC-R3** exhibit inhibitory effects on tumor growth. Among them, INF-α R149 + **R1-AAN-PABC-R3** demonstrates the most significant tumor growth suppression.

### Example 8. Antitumor effects of activated INF-α conjugate in a tumor microenvironment

MC38 cells were subcutaneously implanted into C57BL/6 mice (provided by Shanghai Model Organisms Center), with 2 × 10⁶ cells per mouse. After one week, mice implanted with MC38 tumors were randomly divided into 4 groups.

The groups are as follows:
Group 1 (G1): Injected with 100 µg of INF-α three times per week;
Group 2 (G2): Injected with 300 µg of INF-α R149 + **R1-AAN-PABC-R3** once per week;
Group 3 (G3): Injected with 100 µg of INF-α R149 + **R1-AAN-PABC-R3** once per week;
Group 4 (G4): Injected with the solvent control once per week.

The treatment cycle lasted for 3 weeks. Tumor volumes of the mice were recorded three times weekly. The tumor volume results at the end of the treatment period are presented in Figure 2.

Among them, **R1-AAN-PABC-R3** specifically refers to Linker 10 in Table 3.

The results showed that 300ug INF-αR149+**R1-AAN-PABC-R3** could effectively inhibit the growth of MC38 tumors, wherein one mouse was cured and five mice showed significant inhibition of 80% or higher. Administration of 100ug INF-αR149+**R1-AAN-PABC-R3** once a week has a better tumor inhibition effect than administration of 100ug INF-α three times a week.

This result shows that the conjugate of INF-α can enhance the activity of effector T cells and the enrichment of INF-α in the tumor microenvironment. Consequently, the conjugates exhibit higher efficacy compared to the original INF-α.

### Example 9. Anti-tumor effects of the combination of an IFN-α conjugate with an anti-PD-1 antibody.

CT26 cells were subcutaneously implanted into Balb/c mice (provided by Shanghai Research Center of model organisms), with 2 × 10⁶ cells per mouse. After one week, mice implanted with CT26 tumors were randomly divided into 4 groups.
Group 1 (G1): Injected with 100 µg of INF-α R149 + **R1-AAN-PABC-R3** once per week;
Group 2 (G2): Injected with 100 µg of anti-PD-1 mouse antibody (Sino Biological; Cat: 50124-RP02) twice per week.
Group 3 (G3): Injected with 100 µg of INF-α R149 + **R1-AAN-PABC-R3** once per week; simultaneously injected with 100 µg of anti-PD-1 mouse antibody twice per week;
Group 4 (G4): Injected with the solvent control once per week.

Among them, R1-AAN-PABC-R specifically refers to Linker 10 in Table 3.

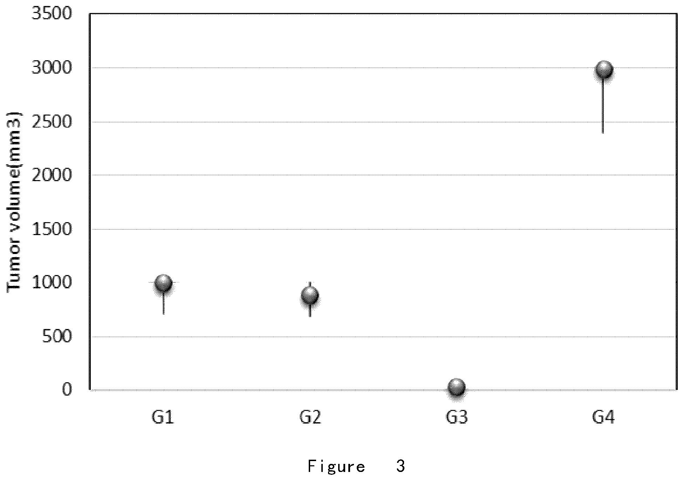

The treatment cycle lasted for 3 weeks. Tumor volumes of the mice were recorded three times weekly. The tumor volume results at the end of the treatment period are presented in Figure 3. The results showed that a combination of 100 µg INF-α R149 + **R1-AAN-PABC-R3** with anti-PD-1 antibody effectively inhibited the growth of CT26 tumors, wherein four mice were cured and all mice exhibited significant tumor suppression. The reduction in tumor volume was extremely pronounced, indicating that the combined use achieved a synergistic enhanced effect.

Therefore, the combination of tumor microenvironment-activated INF-α R149 and PD-1 antibody demonstrated higher efficacy and cure rate.

Above-described examples only show several embodiments of the present disclosure, which are described specifically and in detail. However, it should not be understood as a limiting patent scope of the present disclosure. It should be noted that those skilled in the art can make some adjustments and improvements without departing from the concept of the present disclosure and all these forms are within the scope of protection of the present disclosure. Therefore, the scope of patent protection in the present disclosure should be determined by the appended claims. Simultaneously, each reference provided herein is incorporated by reference to the same extent as if each reference was individually incorporated by reference.

## Claims

1. A biomolecular conjugate of cytokine IFNα with the following structure:
**R1-AAN-PABC-R3-S-Cys-IFNα**
wherein,
Cys represents a cysteine residue introduced through single point mutation on the surface of IFNα;
S represents a sulfur atom on the cysteine residue;
IFNα has an amino acid sequence shown in SEQ ID NO: 1;
R1 is a group that prevents IFNα mutants from binding to its ligand or receptor;
AAN represents a tripeptide, wherein A is alanine, N is aspartic acid and PABC is para-aminobenzyl carbamate;
R3 is a linking arm covalently conjugated to the sulfur atom of the cysteine introduced by the single mutation of IFNα via an EMC group; The function of R3 is to remain attached to the cytokine after AAN cleavage, allowing R3-S-Cys-IFNα partially restores, fully recovers, or even enhances the affinity to the IFNα receptor.

2. The biomolecular conjugate according to claim 1, wherein, R1 is selected from polyethylene glycol with a molecular weight of 44 to 132000, such as polyethylene glycol with a molecular weight of 1000 to 50000, 3,000 to 80000 or 10000 to 60000; or
the IFNα mutant is a mutant obtained by single point mutation on INF-α, wherein the IFNα mutant has mutations of Arg149, Ala145, His57, Gln61, Phe64 or Ser68, Phe27, Ser152, Arg162 residues to Cys, according to the amino acid sequence of SEQ ID NO: 1.

3. The biomolecular conjugate according to claim 2, wherein R1 is selected from:

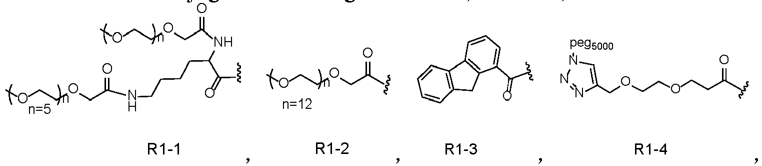

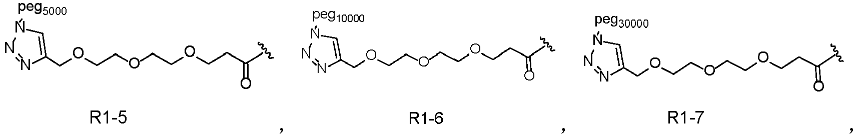

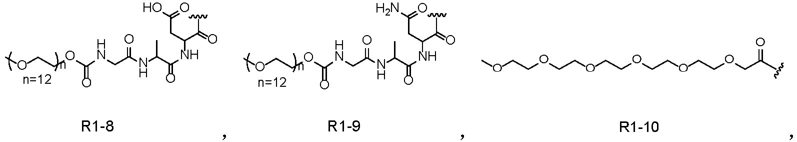

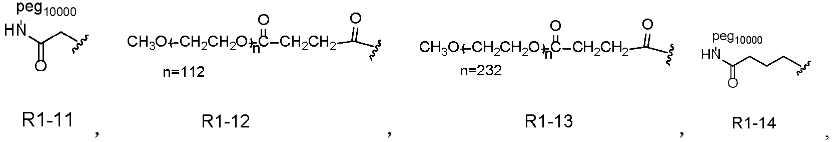

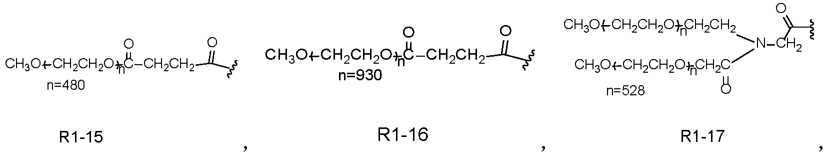

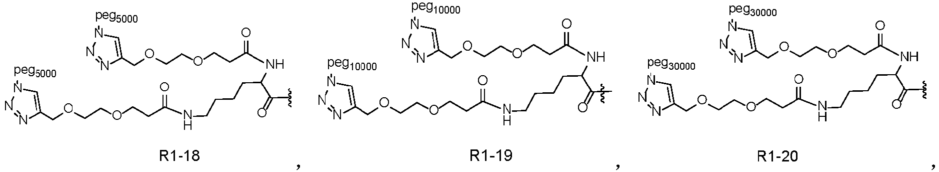

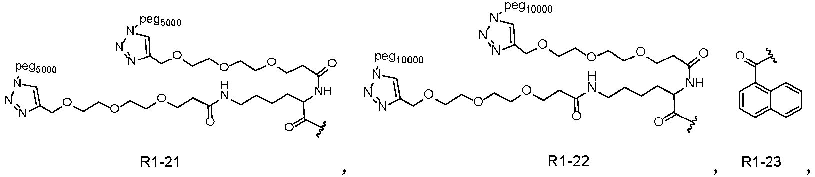

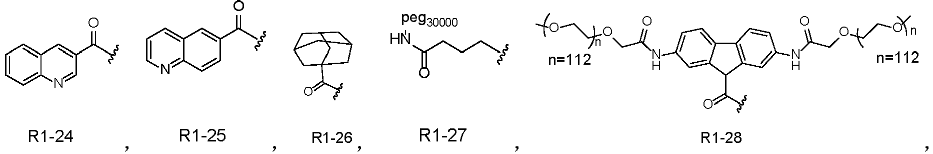

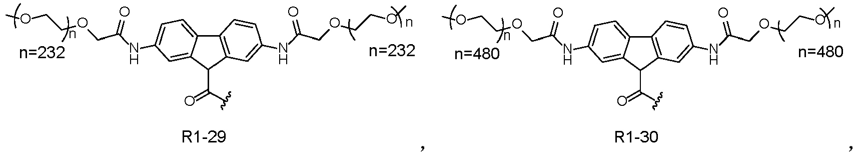

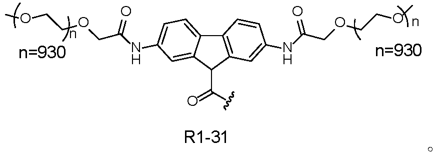

4. The biomolecular conjugate according to claim 1, wherein, R3 is selected from:

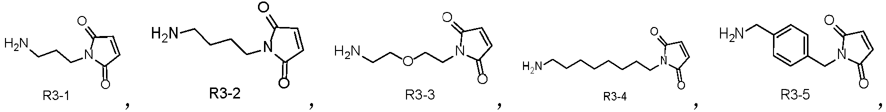

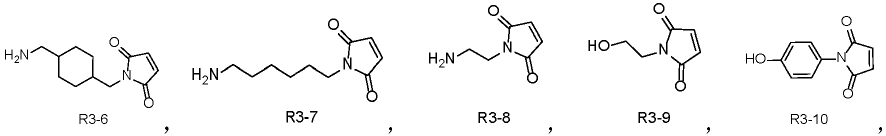

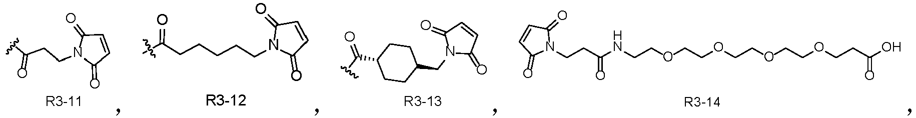

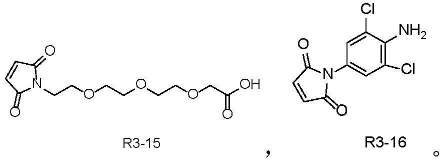

5. The biomolecular conjugate according to claim 1, wherein, **R1-AAN-PABC-R3** has a structure selected from:

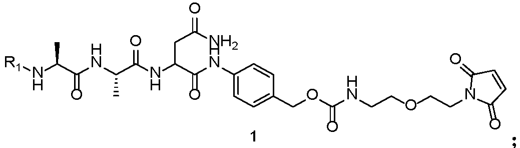

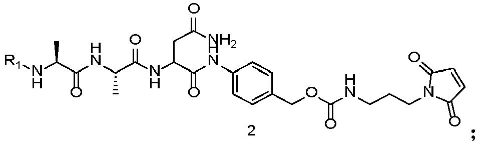

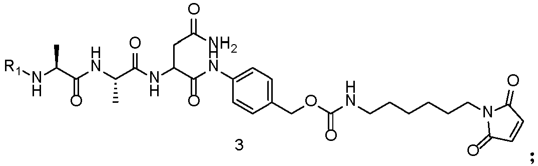

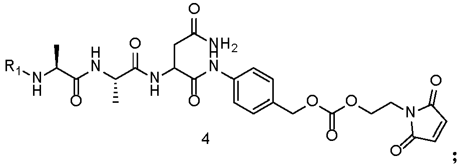

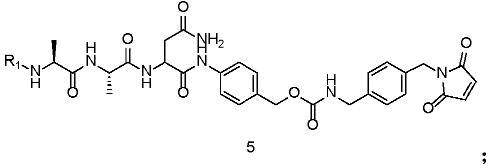

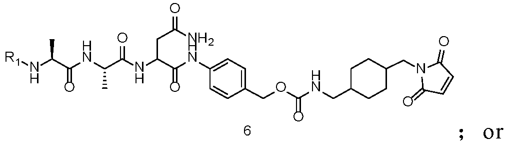

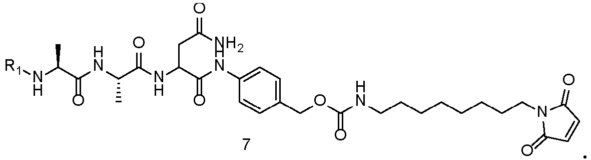

6. A use of the biomolecular conjugate according to any one of claims 1-5, for preparing a composition or drug kit for inhibiting tumors.

7. A use of the biomolecular conjugate according to any one of claims 1-5, for preparing a composition or drug kit for inhibiting tumors in combination with an anti-tumor antibody.

8. A composition or drug kit for inhibiting tumors, wherein the composition or kit comprises the biomolecular conjugate according to any one of claims 1-5; preferably, the composition also comprises a pharmaceutically acceptable carrier.

9. The composition or drug kit according to claim 8, wherein the composition also comprises an anti-tumor antibody; or
the drug kit also comprises: an anti-tumor antibody or a composition comprising an anti-tumor antibody.

10. The composition or drug kit according to claim 9, wherein the anti-tumor antibody is an anti-PD-1 antibody.

11. The composition or drug kit according to claim 8, wherein the biomolecular conjugate and the anti-PD-1 antibody are at a mass ratio of 1: (0.05-50); preferably 1: (0.2-8); more preferably 1: (0.4-5).

12. A method of preparing activated INF-α in a tumor microenvironment, comprising: mutating INF-α at specific residues of the amino acid sequence thereof to Cys by single point mutation.

13. An IFNα mutant, wherein the mutant is obtained by single point mutation on INF-α and wherein the IFNα mutant has mutations of Arg149, Ala145, His57, Gln61, Phe64 or Ser68, Phe27, Ser152, Arg162 residues to Cys, according to the amino acid sequence of SEQ ID NO: 1.

14. An isolated polynucleotide, wherein the polynucleotide encodes the INF-α mutant according to claim 13.

15. A use of the INF-α mutant according to claim 13, for conjugating with R1-AAN-PABC-R3 to form a biomolecular conjugate that inhibits tumors;
wherein,
R1 is a group that prevents IFNα mutants from binding to its ligand or receptor;
AAN represents a tripeptide, wherein A is alanine, N is aspartic acid and PABC is para-aminobenzyl carbamate;
R3 is a linking arm covalently conjugated to the sulfur atom of the cysteine introduced by the single mutation of IFNα via an EMC group; The function of R3 is to remain attached to the cytokine after AAN cleavage, allowing R3-S-Cys-IFNα partially restores, fully recovers, or even enhances the affinity to the IFNα receptor.

16. According to any one of claims 1-15, wherein the tumors comprise: melanoma, NSCLC, head and neck squamous cell carcinoma, urothelial carcinoma, classic Hodgkin lymphoma, gastric cancer, adenocarcinoma of esophagogastric junction, cervical cancer, B-cell lymphoma, hepatocellular carcinoma, Merkel cell carcinoma, renal cell carcinoma, primary liver cancer, small cell lung cancer.
